(19) Europäisches Patentamt
European Patent Office
Office européen des brevets

(11) EP 1 459 764 A1

(12) **EUROPEAN PATENT APPLICATION**
published in accordance with Art. 158(3) EPC

(43) Date of publication:
**22.09.2004 Bulletin 2004/39**

(21) Application number: **02790853.2**

(22) Date of filing: **25.12.2002**

(51) Int Cl.[7]: **A61K 45/00**, A61K 31/135, A61K 38/05, A61K 38/06, A61K 38/08, A61P 25/28, A61P 43/00

(86) International application number:
**PCT/JP2002/013478**

(87) International publication number:
**WO 2003/055521 (10.07.2003 Gazette 2003/28)**

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(30) Priority: **26.12.2001 JP 2001394236**

(71) Applicant: **Takeda Chemical Industries, Ltd.**
**Osaka-shi, Osaka 541-0045 (JP)**

(72) Inventors:
- **MIYAMOTO, Masaomi**
  **Takarazuka-shi, Hyogo 665-0841 (JP)**
- **TAKAHASHI, Hideki**
  **Suita-shi, Osaka 565-0836 (JP)**
- **FUKUMOTO, Hiroaki**
  **Kawabe-gun, Hyogo 666-0257 (JP)**
- **OHKAWA, Shigenori**
  **Takatsuki-shi, Osaka 569-1121 (JP)**

(74) Representative: **Rickard, Timothy Mark Adrian**
**Takeda Patent Office,**
**11-12 Charles II Street**
**London SW1Y 4QU (GB)**

(54) **REMEDIES FOR MILD RECOGNITION DEFLICT**

(57) It is intended to provide agents for treating mild cognitive impairment which comprise compounds having an effect of inhibiting the production, secretion, aggregation, and/or accumulation of β-amyloid proteins, prodrugs thereof or salts of the same and inhibit the progress of mild cognitive impairment to Alzheimer's disease using the same.



Printed by Jouve, 75001 PARIS (FR)

## Description

Technical Field

The present invention relates to an agent for treating mild cognitive impairment.

Background Art

Mild cognitive impairment (MCI) is also referred as mild memory impairment. Patients with MCI are diagnosed as neither normal nor dementia, and their cognitive abilities are at the level between normal and dementia. Generally, patients with MCI have mild memory deficit, mild cognitive deficit, and minimal dysfunction in activities of daily living. According to a recent report, mild cognitive impairment could be regarded as the initial stage of or a transitional phase to Alzheimer's disease (AD) because the conditions of MCI patients steadily progressed to more severe stages of dementia and in many cases, pathological observations in their nerves were similar to those of AD patients (Archive of Neurology, vol.58, pp.397-405 (2001)).

Alzheimer's disease is a neurodegenerative disorder characterized by senile plaque formation and neurofibrillary tangles as well as nerve cell degeneration and loss. The most characteristic feature of Alzheimer's disease is senile plaques, which are formed by deposition of biological constituents in the brain, whose principal component is β-amyloid protein (hereafter, may be simply referred as Aβ) (Biochemical Biophysical Research Communication, vol. 122, pp.1131 (1984)). It is known that Aβ consists of 40 or 42 amino acids (hereafter, simply referred as Aβ40 or Aβ42, respectively) and has a cytotoxic effect on nerve cells (Trend in Neuroscience (TINS), vol.16, pp.409 (1993); Science, vol.274, pp.99 (1996); Nature, vol.395, pp.755 (1998); Neurobiology of Aging, vol.201, pp.20 (1999), etc.).

Thus, drugs for inhibiting production or secretion of Aβ are effective in preventing or treating Alzheimer's disease. An enzyme immunoassay (EIA) technique for Aβ has been established recently, which enables not only screening of compounds inhibiting the secretion of Aβ from neuronal cell culture but also determination of the quantities of Aβ in various biological tissues, blood and cerebrospinal fluid (for example, Science, vol;.264, pp.1336 (1994); Biochemistry, vol.34, pp.10272 (1995); Science, vol.274, pp.99 (1996)).

The precursor protein of Aβ, APP (Amyloid Precursor Protein) is cleaved by the action of β-secretase and γ-secretase to produce Aβ. Some laboratories have reported recently that cDNA of β-secretase was isolated and then identified (for example, Science, vol.286, pp.735 (1999); Nature, vol.402, pp.533 (1999); Nature, vol.402, pp.537 (1999)). It was found that some familial Alzheimer's disease patients have mutation in the APP gene and it was reported that the cells transfected with the mutant genes increased Aβ production and secretion (for example, Nature, vol.360, pp.672 (1992), Science, vol.259, pp.514 (1993); Science, vol.264, pp.1336 (1994)). Accordingly, it is considered that drugs for inhibiting β-secretase can inhibit Aβ production or secretion and therefore are useful for preventing or treating patients who have increased Aβ protein in their brains, such as those who are susceptible to diseases caused by Aβ genetically (for example, Alzheimer's disease and Down's syndrome), in particular, familial Alzheimer's disease, and those who have increased Aβ protein in their brains due to trauma or the like.

Moreover, inhibition of β-secretase leads to blocking or inhibiting a metabolic pathway from APP to Aβ protein, while it leads to accelerating secretion of secretory APP that is produced by the action of α-secretase (hereafter, simply referred as sAPPα). It has been reported that sAPPα has a neurotrophic factor-like activity (Neuron, vol.10, pp.243-254 (1993); Trend in Neuroscience (TINS), vol.16, pp.409 (1993), etc.). Such a neurotrophic factor-like activity includes (1) a nerve cell-survival or - maintenance, (2) an accelerating activity on formation of synapses, (3) a preventing activity on nerve cells death and (4) a long-term potentiation on nerve cells in hippocampus. Thus it is considered that drugs for inhibiting β-secretase can accelerate secretion of sAPPα and therefore are useful for preventing or treating (1) neurodegenerative disease, (2) nervous disorder due to cerebrovascular injury, head trauma, spinal cord injury, sequelae of encephalitis, or cerebral palsy, (3) memory impairment, (4) mental disease, and the like.

However, it has never been demonstrated that active treatment of mild cognitive impairment with recognition that mild cognitive impairment is a kind of disease may inhibit development of Alzheimer's disease.

On the other hand, JP-A 11-80098 discloses an amyloid β-protein production or secretion inhibitor which comprises a compound represented by the formula:

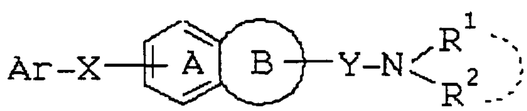

wherein, Ar is an optionally substituted aromatic group, X is (i) a bond, (ii) -S-, -SO- or $-SO_2-$, (iii) $C_{1-6}$ alkylene, $C_{2-6}$

alkenylene or $C_{2-6}$ alkynylene, each of which may be substituted with one to three substituents selected from oxo and $C_{1-6}$ alkyl, (iv) -CO-O- or (v) a group represented by the formula: $-(CH_2)p-X^1-$, $-(CH_2)p-X^1-(CH_2)q-$, $-(CH_2)r-CO-X^1-$, $-SO_2-NR^8-$, or $-(CH_2)r-SO_2-NF^8-$, wherein $X^1$ is an oxygen atom or $NR^8$, $R^8$ is a hydrogen atom, an optionally substituted hydrocarbon group or acyl, p is an integer of 0 to 5, q is an integer of 1 to 5, p+q is an integer of 1 to 5, and r is an integer of 1 to 4; Y is a divalent $C_{1-6}$ aliphatic hydrocarbon group in which an oxygen atom or a sulfur atom may intervene and which may be substituted, $R^1$ and $R^2$ are independently a hydrogen atom or an optionally substituted lower alkyl, or $R^1$ and $R^2$ are taken together with the adjacent nitrogen atom to form an optionally substituted nitrogen-containing heterocyclic ring, ring A is a benzene ring which may be further substituted in addition to the group represented by the formula -X-Ar wherein each symbol is as defined above; and ring B is a 4-to 8-membered ring which may be further substituted in addition to the group represented by the formula $-Y-NR^1R^2$ wherein each symbol is as defined above; or a salt thereof.

JP-A 8-502587 (WO94/10569) discloses a method for identifying a β-amyloid peptide (βAP) production inhibitor.

JP-A 7-165606 discloses a method for identifying a βAP production inhibitor.

JP-A 10-509797 (WO96/15452) discloses a method for detecting a soluble β-amyloid peptide in a liquid sample.

JP-A 11-507538 (W096/40885) discloses a composition comprising isolated and purified enzymes which specifically cleave β-amyloid precursor protein at the β-amyloid peptide cleavage sites.

JP-A 9-178743 discloses a method for determining the quantity of soluble β-amyloid precursor protein (APP) by using an antibody against β-amyloid protein or soluble APP.

Objective of the Invention

The present invention is intended to provide an agent for treatment of mild cognitive impairment and to inhibit the progression of mild cognitive impairment to Alzheimer's disease.

Summary of the Invention

The present inventors made their efforts at establishing treatment of mild cognitive impairment and as a result, found that compounds having an inhibitory effect on production, secretion, aggregation and/or accumulation of β-amyloid protein are useful for treating mild cognitive impairment and inhibiting the progression of MCI to Alzheimer's disease, and finally completed the present invention.

Namely, the present invention provides:

(1) a agent for preventing or treating mild cognitive impairment which comprises a compound having an inhibitory effect on production, secretion, aggregation and/or accumulation of β-amyloid protein or a prodrug thereof;

(2) the agent according to the above (1) wherein the compound is a compound having an inhibitory effect on β-secretase;

(3) the agent according to the above (1) wherein the compound is a compound represented by the formula:

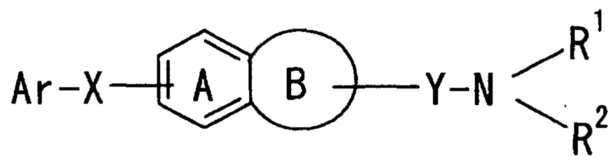

wherein, Ar is an optionally substituted aromatic group, X is a divalent $C_{1-6}$ aliphatic hydrocarbon group which may contain one or two divalent groups selected from -O-, -S-, -CO-, -SO-, $-SO_2-$ and -COO-, Y is a divalent $C_{1-6}$ aliphatic hydrocarbon group, $R^1$ and $R^2$ may be the same or different and each is a hydrogen atom or optionally substituted $C_{1-6}$ alkyl, ring A is a benzene ring which may have an additional substituent, and ring B is a 4- to 8-membered ring which may have an additional substituent, or a salt or hydrate thereof;

(4) the agent according to the above (3) wherein the compound is selected from 6-(4-biphenylyl)methoxy-2-[2-(N, N-dimethylamino)ethyl)tetralin, 6-(4-biphenylyl)methoxy-2-(N,N-dimethylamino)methyltetralin, 6-(4-biphenylyl) methoxy-2-(N,N-dipropylamino)methyltetralin, 2-(N,N-dimethylamino)methyl-6-(4'-methoxybiphenyl-4-yl)methoxytetralin, 6-(4-biphenylyl)methoxy-2-[2-(N,N-diethylamino)ethyl]tetralin, 2-[2-(N,N-dimethylamino)ethyl]-6-(4'-methylbiphenyl-4-yl)methoxytetralin, 2-[2-(N,N-dimethylamino)ethyl]-6-(4'-methoxybiphenyl-4-yl)methoxytetralin,

6-(2',4'-dimethoxybiphenyl-4-yl)methoxy-2-[2-(N,N-dimethylamino)ethyl]tetralin, 6-[4-(1,3-benzodioxol-5-yl)phenyl]methoxy-2-[2-(N,N-dimethylamino)ethyl]tetralin, 6-(3',4'-dimethoxybiphenyl-4-yl)methoxy-2-(2-(N,N-dimethylamino)ethyl]tetralin, and optical active forms, salts and hydrates thereof;

(5) the agent according to the above (2) wherein the compound having an inhibitory effect on β-secretase is OM99-2;

(6) the agent according to the above (1) wherein the compound is a compound having an inhibitory effect on γ-secretase;

(7) the agent according to the above (1) wherein the compound is a compound having an inhibitory effect on aggregation of β-amyloid protein;

(8) the agent according to the above (7) wherein the compound having an inhibitory effect on aggregation of β-amyloid protein is PTI-00703, ALZHEMED (NC-531), PPI-368, PPI-558 or SKF-74652;

(9) an agent for inhibiting the progression of mild cognitive impairment to Alzheimer's disease, which comprises a compound having an inhibitory effect on production, secretion, aggregation and/or accumulation of β-amyloid protein or a prodrug thereof;

(10) a method for preventing or treating mild cognitive impairment, which comprises administering to a mammal an effective amount of a compound having an inhibitory effect on production, secretion, aggregation and/or accumulation of β-amyloid protein or a prodrug thereof;

(11) a method for inhibiting the progression of mild cognitive impairment to Alzheimer's disease, which comprises administering to a mammal an effective amount of a compound having an inhibitory effect on production, secretion, aggregation and/or accumulation of β-amyloid protein or a prodrug thereof;

(12) use of a compound having an inhibitory effect on production, secretion, aggregation and/or accumulation of β-amyloid protein or a prodrug thereof, for the manufacture of an agent for preventing or treating mild cognitive impairment; and

(13) use of a compound having an inhibitory effect on production, secretion, aggregation and/or accumulation of β-amyloid protein or a prodrug thereof, for the manufacture of an agent for inhibiting the progression of mild cognitive impairment to Alzheimer's disease.

Brief Description of Drawings

Fig. 1 is a graph showing an ameliorative effect on learning deficit in old rats observed in Experimental Example 1.

Fig. 2 is a graph showing an inhibiting effect of the compound A on accumulation of insoluble Aβ40 (Fig. 2A) and Aβ42 (Fig. 2B) in transgenic mice with Swedish APP mutation in Experimental Example 2. ##P<0.01 (compared with that of a group of 8-month old mice, Student's t-test), *P<0.05 (compared with that of a control group of 13- to 14-month old mice, Student's t-test).

Fig. 3 is a graph showing an inhibiting effect of the compound A on the area (Fig. 3A) and the number (Fig. 3B) of amyloid plaques in transgenic mice with Swedish APP mutation in Experimental Example 2. *P<0.01 (compared with that of a control group, Student's t-test)

Detailed Description of the Invention

The term "mild cognitive impairment", as used herein, means impairment whose clinical condition is on a level of 0.5 according to clinical dementia rating (CDR: subjects are assessed for six categories such as memory, orientation, judgment and the like using 5-stage rating, 0 (normal) to 3 (severe), and based on the result, the final score is calculated using a given algorithm) described in Neurology, vol.43, pp.2412-2414, 1993 (see mentioned-above Archive of Neurology, vol.58. pp.397-405 (2001)).

The compounds having an inhibitory effect on production, secretion, aggregation and/or accumulation of β-amyloid protein used in the present invention include compounds having an inhibitory effect on β-secretase, compounds having an inhibitory effect on γ-secretase and compounds having an inhibitory effect on accumulation of β protein.

The compounds having an inhibitory effect on β-secretase include compounds represented by the above mentioned formula (I) (hereafter, simply referred as compound (I)), or salts or prodrugs thereof.

In the compound (I), Ar indicates an optionally substituted aromatic group.

The "aromatic group" of the "optionally substituted aromatic group" represented by Ar includes a monocyclic aromatic group, a ring-assembly aromatic group and a fused aromatic group.

The "monocyclic aromatic group" includes a monovalent group which is formed by removing any one of hydrogen atoms from a benzene ring or a 5- or 6-membered aromatic heterocycle.

The "5- or 6-membered aromatic heterocycle" includes a 5- or 6-membered aromatic heterocycle containing

one or more (for example, 1 to 3 and preferably 1 or 2) heteroatoms selected from a nitrogen atom, a sulfur atom and an oxygen atom, in addition to carbon atoms. Specific examples thereof are thiophene, furan, pyrrole, imidazole, pyrazole, thiazole, oxazole, pyridine, pyrazine, pyrimidine, pyridazine ring and the like.

Specific examples of the monocyclic aromatic group mentioned above include phenyl, 2- or 3-thienyl, 2- or 3-furyl, 1-, 2- or 3-pyrrolyl, 2- or 4-imidazolyl, 3- or 4-pyrazolyl, 2-, 4- or 5-thiazolyl, 2-, 4- or 5-oxazolyl, 2-, 3- or 4-pyridyl, 2-pyrazinyl, 2-, 4- or 5-pyrimidinyl, and 3- or 4-pyridazinyl and in particular, phenyl is preferable among them.

The "ring-assembly aromatic group" includes a group formed by removing any one of hydrogen atoms from an aromatic ring assembly in which two or more (preferably, 2 or 3) aromatic rings are directly bound through single bonds and in which the number of the single bonds through which the rings are directly bound is one less than the number of the rings. The "aromatic ring" includes aromatic hydrocarbon and an aromatic heterocycle.

The "aromatic hydrocarbon" includes $C_{6-14}$ monocyclic or fused polycyclic (for example, dicyclic or tricyclic) aromatic hydrocarbon (for example, benzene, naphthalene, indene, or anthracene).

The "aromatic heterocycle" include 5- to 14-membered, preferably 5- to 10-membered aromatic heterocycles containing one or more (for example, one to four and preferably, one to two) heteroatoms selected form a nitrogen atom, a sulfur atom and an oxygen atom, in addition to carbon atoms. Specific examples thereof include aromatic heterocycles such as thiophene, benzothiophene, benzofuran, benzimidazole, benzoxazole, benzothiazole, benzisothiazole, naphtho[2,3-b]thiophene, furan, phenoxathiin, pyrrole, imidazole, pyrazole, oxazole, isoxazole, 1,2,4-oxadiazole, 1,3,4-oxadiazole, 1,2,4-thiadiazole, 1,3,4-thiadiazole, pyridine, pyrazine, pyrimidine, pyridazine, indole, isoindole, 1H-indazole, purine, 4H-quinolizine, isoquinoline, quinoline, phthalazine, naphthylidine, quinoxaline, quinazoline, cinnoline, carbazole, β-carboline, phenanthridine, acridine, phenazine, thiazole, isothiazole, phenothiazine, furazan, phenoxazine, phthalimide, 2-, 3- or 4-pyridone, or 2-, 3-or 4-quinolone, and fused rings of the above mentioned aromatic heterocycle (preferably monocycle) with one or more (preferably, one or two) aromatic rings (for example, a benzene ring).

As the aromatic ring assembly in which aromatic rings are directly bound through single bonds, for example, an aromatic ring assembly consisting of two or three (preferably, two) rings selected from a benzene ring, a naphthalene ring and 5- to 10-membered (preferably, 5- or 6-membered) aromatic heterocycles may be used. Preferable examples of the aromatic ring assembly include an aromatic ring assembly consisting of two or three aromatic rings selected from benzene, naphthalene, pyridine, pyrimidine, thiophene, furan, thiazole, isothiazole, oxazole, 1,2,4-oxadiazole, 1,3,4-oxadiazole, 1,2,4-thiadiazole, 1,3,4-thiadiazole, quinoline, isoquinoline, indole, benzothiophene, benzoxazole, benzothiazole and benzofuran. More specific examples thereof include 2-, 3- or 4-biphenylyl, 3-(1-naphthyl)-1,2,4-oxadiazol-5-yl, 3-(2-naphthyl)-1,2,4-oxadiazol-5-yl, 3-(2-benzofuranyl)-1,2,4-oxadiazol-5-yl, 3-phenyl-1,2,4-oxadiazol-5-yl, 3-(2-benzoxazolyl)-1,2,4-oxadiazol-2-yl, 3-(3-indolyl)-1,2,4-oxadiazol-2-yl, 3-(2-indolyl)-1,2,4-oxadiazol-2-yl, 4-phenylthiazol-2-yl, 4-(2-benzofuranyl)thiazol-2-yl, 4-phenyl-1,3-oxazol-5-yl, 5-phenylisothiazol-4-yl, 5-phenyloxazol-2-yl, 4-(2-thienyl)phenyl, 4-(3-thienyl)phenyl, 3-(3-pyridyl)phenyl, 4-(3-pyridyl)phenyl, 6-phenyl-3-pyridyl, 5-phenyl-1,3,4-oxadiazol-2-yl, 4-(2-naphthyl)phenyl, 4-(2-benzofuranyl)phenyl and 4,4'-terphenyl, and among them, biphenylyl (2-, 3- or 4-biphenylyl) is preferable.

The "fused aromatic group" includes a monovalent group formed by removing any one of hydrogen atoms from a fused polycyclic (preferably, dicyclic to tetracyclic and preferably, dicyclic or tricyclic) aromatic ring. The "fused polycyclic aromatic ring" includes a fused polycyclic aromatic hydrocarbon and a fused polycyclic aromatic heterocycle.

The "fused polycyclic aromatic hydrocarbon" includes a fused polycyclic (dicyclic or tricyclic) aromatic $C_{9-14}$ hydrocarbon (for example, naphthalene, indene, anthracene).

The "fused polycyclic aromatic heterocycle" includes a 9- to 14-membered, preferably 9- or 10-membered fused polycyclic aromatic heterocycle containing one or more (for example, one to four) heteroatoms selected from a nitrogen atom, a sulfur atom and an oxygen atom, in addition to carbon atoms. Specific examples thereof include aromatic heterocycles such as benzofuran, benzimidazole, benzoxazole, benzothiazole, benzisothiazole, naphtho [2,3-b]thiophene, isoquinoline, quinoline, indole, quinoxaline, phenanthridine, phenothiazine, phenoxazine and phthalimide.

Specific examples of the above-mentioned fused aromatic group include 1-naphthyl, 2-naphthyl, 2-quinolyl, 3-quinolyl, 4-quinolyl, 2-benzofuranyl, 2-benzothiazolyl, 2-benzimidazolyl, 1-indolyl, 2-indolyl and 3-indolyl, and among them, 1-naphthyl and 2-naphthyl are preferable.

A substituent for the aromatic group represented by Ar includes halogen atoms (for example, fluorine, chlorine, bromine, or iodine), $C_{1-3}$ alkylenedioxy (for example, methylenedioxy or ethylenedioxy), nitro, cyano, optionally halogenated $C_{1-6}$ alkyl, $C_{6-10}$ aryloxy-$C_{1-6}$ alkyl (for example, phenoxymethyl), $C_{1-6}$ alkyl-$C_{6-10}$ aryl-$C_{2-6}$ alkenyl (for example, methylphenylethenyl), optionally halogenated $C_{3-6}$ cycloalkyl, optionally substituted $C_{7-16}$ aralkyl, optionally halogenated $C_{1-6}$ alkoxy, optionally halogenated $C_{1-6}$ alkylthio, hydroxy, optionally substituted $C_{6-10}$ aryloxy, $C_{6-10}$ aryl-$C_{7-16}$ aralkyloxy (for example, phenylbenzyloxy), amino, mono-$C_{1-6}$ alkylamino (for example, methylamino, ethylamino, propylamino, isopropylamino, or butylamino), di-$C_{1-6}$ alkylamino (for example, dimethylamino, diethylamino, dipropylamino, dibutylamino, or ethylmethylamino), optionally substituted 5- to 7-membered saturated cyclic amino, acyl, acylamino

and acyloxy. Said "aromatic group" may have, for example, one to five (preferably, one to three) of the above-mentioned substituents at the substitutable positions, and if it has two or more substituents, the substituents may be the same or different from each other.

The "'$C_{7-16}$ aralkyl" of the "optionally substituted $C_{7-16}$ aralkyl" among the above mentioned substituents for the aromatic groups represented by Ar, includes benzyl, phenethyl and naphthylmethyl.

The "$C_{6-10}$ aryloxy" of the "optionally substituted $C_{6-10}$ aryloxy" includes phenyloxy and naphthyloxy. A "substituent" for these "optionally substituted $C_{7-16}$ aralkyl" and "optionally substituted $C_{6-10}$ aryloxy" includes halogen atoms (for example, fluorine, chlorine, bromine, or iodine), $C_{1-3}$ alkylenedioxy (for example, methylenedioxy or ethylenedioxy), nitro, cyano, optionally halogenated $C_{1-6}$ alkyl, optionally halogenated $C_{3-6}$ cycloalkyl, optionally halogenated $C_{1-6}$ alkoxy, optionally halogenated $C_{1-6}$ alkylthio, hydroxy, amino, mono-$C_{1-6}$ alkylamino (for example, methylamino, ethylamino, propylamino, isopropylamino, or butylamino), di-$C_{1-6}$ alkylamino (for example, dimethylamino, diethylamino, dipropylamino, dibutylamino, or ethylamino), formyl, carboxy, carbamoyl, optionally halogenated $C_{1-6}$ alkyl-carbonyl, $C_{1-6}$ alkoxy-carbonyl (for example, methoxycarbonyl, ethoxycarbonyl, propoxycarbonyl, or tert-butoxycarbonyl), mono-$C_{1-6}$ alkyl-carbamoyl (for example, methylcarbamoyl, or ethylcarbamoyl), di-$C_{1-6}$ alkyl-carbamoyl (for example, dimethylcarbamoyl, diethylcarbamoyl, or ethylmethylcarbamoyl), optionally halogenated $C_{1-6}$ alkylsulfonyl, formylamino, optionally halogenated $C_{1-6}$ alkyl-carboxamido, $C_{1-6}$ alkoxy-carboxamido (for example, methoxycarboxamido, ethoxycarboxamido, propoxycarboxamido, or butoxycarboxamido), $C_{1-6}$ alkylsulfonylamino (for example, methylsulfonylamino, or ethylsulfonylamino), $C_{1-6}$ alkyl-carbonyloxy (for example, acetoxy, or propanoyloxy), $C_{1-6}$ alkoxy-carbonyloxy (for example, methoxycarbonyloxy, ethoxycarbonyloxy, propoxycarbonyloxy, or butoxycarbonyloxy) , mono-$C_{1-6}$ alkyl-carbamoyloxy (for example, methylcarbamoyloxy or ethylcarbamoyloxy), and di-$C_{1-6}$ alkyl-carbamoyloxy (for example, dimethylcarbamoyloxy or diethylcarbamoyloxy) , and one to five of these substituents may be used.

The "5- to 7-membered saturated cyclic amino" of the "optionally substituted 5- to 7-membered saturated cyclic amino" among the above mentioned substituents for the aromatic groups represented by Ar, includes morpholino, thiomorpholino, piperazin-1-yl, piperidino, pyrrolidin-1-yl and hexamethylen-1-yl. A "substituent" for the "optionally substituted 5- or 7-membered saturated cyclic amino" includes optionally halogenated $C_{1-6}$ alkyl, optionally substituted $C_{6-14}$ aryl, optionally substituted $C_{7-19}$ aralkyl, optionally substituted 5- to 10-membered aromatic heterocyclic groups, optionally substituted $C_{6-10}$ arylcarbonyl, optionally halogenated $C_{1-6}$ alkyl-carbonyl and optionally halogenated $C_{1-6}$ alkylsulfonyl, and one to three of these substituents may be used.

Here, the "$C_{6-14}$ aryl" of the "optionally substituted $C_{6-14}$ aryl" includes phenyl, 1-naphthyl, 2-naphthyl, 2-indenyl and 2-anthryl. Among them phenyl is preferable. The "$C_{7-19}$ aralkyl" of the "optionally substituted $C_{7-19}$ aralkyl" includes benzyl, phenethyl, diphenylmethyl, triphenylmethyl, 1-naphthylmethyl, 2-naphthylmethyl, 2,2-diphenylethyl, 3-phenylpropyl, 4-phenylbutyl and 5-phenylpentyl, and benzyl is preferable among them. The "5-to 10-membered aromatic heterocyclic group" of the "optionally substituted 5- to 10-membered aromatic heterocyclic group" includes 2-, 3- or 4-pyridyl, 1-, 2- or 3-indolyl and 2- or 3-thienyl, and 2-, 3- or 4-pyridyl is preferable among them. The "$C_{6-10}$ arylcarbonyl" of the "optionally substituted $C_{6-10}$ aryl-carbonyl" includes benzoyl, 1-naphthoyl and 2-naphthoyl. A "substituent" for these "optionally substituted $C_{6-14}$ aryl", "optionally substituted $C_{7-19}$ aralkyl", "optionally substituted 5- to 10-membered aromatic heterocyclic group" and "optionally substituted $C_{6-10}$ aryl-carbonyl" respectively include halogen atoms (for example, fluorine, chlorine, bromine, or iodine), $C_{1-3}$ alkylenedioxy (for example, methylenedioxy, or ethylenedioxy), nitro, cyano, optionally halogenated $C_{1-6}$ alkyl, optionally halogenated $C_{3-6}$ cycloalkyl, optionally halogenated $C_{1-6}$ alkoxy, optionally halogenated $C_{1-6}$ alkylthio, hydroxyl, amino, mono-$C_{1-6}$ alkylamino (for example, methylamino, ethylamino, propylamino, isopropylamino, or butylamino), di-$C_{1-6}$ alkylamino (for example, dimethylamino, diethylamino, dipropylamino, dibutylamino, or ethylmethylamino), formyl, carboxy, carbamoyl, optionally halogenated $C_{1-6}$ alkyl-carbonyl, $C_{1-6}$ alkoxy-carbonyl (for example, methoxycarbonyl, ethoxycarbonyl, propoxycarbonyl, or tert-butoxycarbonyl), mono-$C_{1-6}$ alkyl-carbamoyl (for example, methylcarbamoyl or ethylcarbamoyl), di-$C_{1-6}$ alkyl-carbamoyl (for example, dimethylcarbamoyl, diethylcarbamoyl, or ethylmethylcarbamoyl), optionally halogenated $C_{1-6}$ alkylsulfonyl, formylamino, optionally halogenated $C_{1-6}$ alkyl-carboxamido, $C_{1-6}$ alkoxy-carboxamido (for example, methoxycarboxamido, ethoxycarboxamido, propoxycarboxamido, or butoxycarboxamido), $C_{1-6}$ alkylsulfonylamino (for example, methylsulfonylamino or ethylsulfonylamino), $C_{1-6}$ alkyl-carbonyloxy (for example, acetoxy or propanoyloxy), $C_{1-6}$ alkoxy-carbonyloxy (for example, methoxycarbonyloxy, ethoxycarbonyloxy, propoxycarbonyloxy, or butoxycarbonyloxy), mono-$C_{1-6}$ alkyl-carbamoyloxy (for example, methylcarbamoyloxy or ethylcarbamoyloxy), and di-$C_{1-6}$ alkylcarbamoyloxy (for example, dimethylcarbamoyloxy or diethylcarbamoyloxy), and one to five of these substituents may be used.

In the "acyl", "acylamino" and "acyloxy" exemplified above as the "substituent" for the "optionally substituted aromatic group" represented by Ar, "acyl" includes acyl groups represented by the formula: $-CO-R^a$, $-CO-OR^a$, $-CO-NR^a-R^a$, $-CS-NHR^a$, $-SO_2-R^{aa}$, or $-SO-R^{aa}$

wherein, $R^a$ is (i) a hydrogen atom,

(ii) an optionally substituted hydrocarbon group, specifically a hydrocarbon group which may be substituted with

one to five substituents selected from halogen atoms, $C_{1-3}$ alkylenedioxy, nitro, cyano, optionally halogenated $C_{1-6}$ alkyl, optionally halogenated $C_{3-6}$ cycloalkyl, optionally halogenated $C_{1-6}$ alkoxy, optionally halogenated $C_{1-6}$ alkylthio, hydroxy, amino, mono-$C_{1-6}$ alkylamino, di-$C_{1-6}$ alkylamino, optionally substituted 5- to 7-membered cyclic amino, formyl, carboxy, carbamoyl, optionally halogenated $C_{1-6}$ alkyl-carbonyl, $C_{1-6}$ alkoxy-carbonyl, $C_{6-10}$ aryl-carbonyl, $C_{6-10}$ aryloxy-carbonyl, $C_{7-16}$ aralkyloxy-carbonyl, mono-$C_{1-6}$ alkyl-carbamoyl, di-$C_{1-6}$ alkyl-carbamoyl, $C_{6-10}$ aryl-carbamoyl, optionally halogenated $C_{1-6}$ alkylsulfonyl, $C_{6-10}$ arylsulfonyl, formylamino, optionally halogenated $C_{1-6}$ alkyl-carboxamido, $C_{6-10}$ aryl-carboxamido, $C_{1-6}$ alkoxy-carboxamido, $C_{1-6}$ alkylsulfonylamino, $C_{1-6}$ alkyl-carbonyloxy, $C_{6-10}$ aryl-carbonyloxy, $C_{1-6}$ alkoxy-carbonyloxy, mono-$C_{1-6}$ alkyl-carbamoyloxy, di-$C_{1-6}$ alkyl-carbamoyloxy, $C_{6-10}$ aryl-carbamoyloxy, nicotinoyloxy and $C_{6-10}$ aryloxy, or

(iii) an optionally substituted heterocyclic group, specifically a heterocyclic group which may be substituted with one to five substituents selected from halogen atoms, $C_{1-3}$ alkylenedioxy, nitro, cyano, optionally halogenated $C_{1-6}$ alkyl, optionally halogenated $C_{3-6}$ cycloalkyl, optionally halogenated $C_{1-6}$ alkoxy, optionally halogenated $C_{1-6}$ alkylthio, hydroxy, amino, mono-$C_{1-6}$ alkylamino, di-$C_{1-6}$ alkylamino, optionally substituted 5- to 7-membered cyclic amino, formyl, carboxy, carbamoyl, optionally halogenated $C_{1-6}$ alkyl-carbonyl, $C_{1-6}$ alkoxy-carbonyl, $C_{6-10}$ aryl-carbonyl, $C_{6-10}$ aryloxy-carbonyl, $C_{7-16}$ aralkyloxy-carbonyl, mono-$C_{1-6}$ alkyl-carbamoyl, di-$C_{1-6}$ alkyl-carbamoyl, $C_{6-10}$ aryl-carbamoyl, optionally halogenated $C_{1-6}$ alkylsulfonyl, $C_{6-10}$ arylsulfonyl, formylamino, optionally halogenated $C_{1-6}$ alkyl-carboxamido, $C_{6-10}$ aryl-carboxamido, $C_{1-6}$ alkoxy-carboxamido, $C_{1-6}$ alkylsulfonylamino, $C_{1-6}$ alkyl-carbonyloxy, $C_{6-10}$ aryl-carbonyloxy, $C_{1-6}$ alkoxy-carbonyloxy, mono-$C_{1-6}$ alkyl-carbamoyloxy, di-$C_{1-6}$ alkyl-carbamoyloxy, $C_{6-10}$ aryl-carbamoyloxy, nicotinoyloxy and $C_{6-10}$ aryloxy,

$R^{aa}$ is (i) an optionally substituted hydrocarbon group, specifically a hydrocarbon group which may be substituted with one to five substituents selected from halogen atoms, $C_{1-3}$ alkylenedioxy, nitro, cyano, optionally halogenated $C_{1-6}$ alkyl, optionally halogenated $C_{3-6}$ cycloalkyl, optionally halogenated $C_{1-6}$ alkoxy, optionally halogenated $C_{1-6}$ alkylthio, hydroxy, amino, mono-$C_{1-6}$ alkylamino, di-$C_{1-6}$ alkylamino, optionally substituted 5- to 7-membered cyclic amino, formyl, carboxy, carbamoyl, optionally halogenated $C_{1-6}$ alkyl-carbonyl, $C_{1-6}$ alkoxy-carbonyl, $C_{6-10}$ aryl-carbonyl, $C_{6-10}$ aryloxy-carbonyl, $C_{7-16}$ aralkyloxy-carbonyl, mono-$C_{1-6}$ alkyl-carbamoyl, di-$C_{1-6}$ alkyl-carbamoyl, $C_{6-10}$ aryl-carbamoyl, optionally halogenated $C_{1-6}$ alkylsulfonyl, $C_{6-10}$ arylsulfonyl, formylamino, optionally halogenated $C_{1-6}$ alkyl-carboxamido, $C_{6-10}$ aryl-carboxamido, $C_{1-6}$ alkoxy-carboxamido, $C_{1-6}$ alkylsulfonylamino, $C_{1-6}$ alkyl-carbonyloxy, $C_{6-10}$ aryl-carbonyloxy, $C_{1-6}$ alkoxy-carbonyloxy, mono-$C_{1-6}$ alkyl-carbamoyloxy, di-$C_{1-6}$ alkyl-carbamoyloxy, $C_{6-10}$ aryl-carbamoyloxy, nicotinoyloxy and $C_{6-10}$ aryloxy, or

(ii) an optionally substituted heterocyclic group, specifically a heterocyclic group which may be substituted with one to five substituents selected from halogen atoms, $C_{1-3}$ alkylenedioxy, nitro, cyano, optionally halogenated $C_{1-6}$ alkyl, optionally halogenated $C_{3-6}$ cycloalkyl, optionally halogenated $C_{1-6}$ alkoxy, optionally halogenated $C_{1-6}$ alkylthio, hydroxy, amino, mono-$C_{1-6}$ alkylamino, di-$C_{1-6}$ alkylamino, optionally substituted 5- to 7-membered cyclic amino, formyl, carboxy, carbamoyl, optionally halogenated $C_{1-6}$ alkyl-carbonyl, $C_{1-6}$ alkoxy-carbonyl, $C_{6-10}$ aryl-carbonyl, $C_{6-10}$ aryloxy-carbonyl, $C_{7-16}$ aralkyloxy-carbonyl, mono-$C_{1-6}$ alkyl-carbamoyl, di-$C_{1-6}$ alkyl-carbamoyl, $C_{6-10}$ aryl-carbamoyl, optionally halogenated $C_{1-6}$ alkylsulfonyl, $C_{6-10}$ arylsulfonyl, formylamino, optionally halogenated $C_{1-6}$ alkyl-carboxamido, $C_{6-10}$ aryl-carboxamido, $C_{1-6}$ alkoxy-carboxamido, $C_{1-6}$ alkylsulfonylamino, $C_{1-6}$ alkyl-carbonyloxy, $C_{6-10}$ aryl-carbonyloxy, $C_{1-6}$ alkoxy-carbonyloxy, mono-$C_{1-6}$ alkyl-carbamoyloxy, di-$C_{1-6}$ alkyl-carbamoyloxy, $C_{6-10}$ aryl-carbamoyloxy, nicotinoyloxy and $C_{6-10}$ aryloxy, and

$R^b$ is a hydrogen atom or $C_{1-6}$ alkyl, or $R^a$ and $R^b$ may be taken together with the adjacent nitrogen atom to form a nitrogen-containing heterocyclic ring.

Examples of the "optionally substituted 5- to 7-membered saturated cyclic amino" exemplified as a substituent for $R^a$ and $R^{aa}$ are the same as those mentioned above.

The hydrocarbon groups represented by $R^a$ and $R^{aa}$ include groups formed by removing one hydrogen atom from a hydrocarbon compound, and may be, for example, chain or cyclic hydrocarbon groups (for example, alkyl, alkenyl, alkynyl, cycloalkyl, aryl, or aralkyl). Among them, the following $C_{1-19}$ chain or cyclic hydrocarbon groups are preferable:

a) $C_{1-6}$ alkyl (for example, methyl, ethyl, propyl, isopropyl, butyl, isobutyl, sec-butyl, tert-butyl, pentyl, or hexyl),
b) $C_{2-6}$ alkenyl (for example, vinyl, allyl, isopropenyl, or 2-butenyl),
c) $C_{2-6}$ alkynyl (for example, ethynyl, propargyl, or 2-butynyl),
d) $C_{3-6}$ cycloalkyl (for example, cyclopropyl, cyclobutyl, cyclopentyl, or cyclohexyl), wherein said $C_{3-6}$ cycloalkyl may be fused with one benzene ring,
e) $C_{6-14}$ aryl (for example, phenyl, 1-naphthyl, 2-naphthyl, 2-indenyl, or 2-anthryl), preferably phenyl,
f) $C_{7-19}$ aralkyl (for example, benzyl, phenethyl, diphenylmethyl, triphenylmethyl, 1-naphthylmethyl, 2-naphthylmethyl, 2,2-diphenylethyl, 3-phenylpropyl, 4-phenylbutyl, or 5-phenylpentyl), preferably benzyl.

The heterocyclic groups represented by $R^a$ and $R^{aa}$ include monovalent groups formed by removing any one hydrogen atom from a 5- to 14-membered (monocyclic, dicyclic, or tricyclic) heterocyclic ring containing one to four (preferably, one to three) of one or two kinds of heteroatoms selected from a nitrogen atom, a sulfur atom and an oxygen atom, in addition to carbon atoms, preferably (i) a 5- to 14-membered (preferably, 5- to 10-membered) aromatic heterocyclic ring, (ii) a 5- to 10-membered nonaromatic heterocyclic ring or (iii) a 7- to 10-membered bridged heterocyclic ring.

The "5- to 14-membered (preferably, 5- to 10-membered) aromatic heterocyclic ring" mentioned above includes aromatic heterocycles such as thiophene, benzothiophene, benzofuran, benzimidazole, benzoxazole, benzothiazole, benzisothiazole, naphtho[2,3-b]thiophene, furan, phenoxathiin, pyrrole, imidazole, pyrazole, oxazole, 1,2,4-oxadiazole, 1,3,4-oxadiazole, 1,2,4-thiadiazole, 1,3,4-thiadiazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, isoxazole, furazan, phenoxazine or phthalimide, and fused rings of the above-mentioned aromatic heterocycle (preferably monocycles) with one or more (preferably, one or two) aromatic rings (for example, a benzene ring).

The "5- to 10-membered nonaromatic heterocyclic ring" mentioned above includes pyrrolidine, imidazoline, pyrazolidine, pyrazoline, piperidine, piperazine, morpholine and thiomorpholine.

The "7- to 10-membered bridged heterocyclic ring" mentioned above includes quinuclidine and 7-azabicyclo [2.2.1]heptane.

The "heterocyclic group" is preferably a 5- to 10-membered (monocyclic or bicyclic) heterocyclic group containing one to four of one or two kinds of heteroatoms selected from a nitrogen atom, a sulfur atom and an oxygen atom, in addition to carbon atoms. Specific examples thereof include aromatic heterocyclic groups such as 2- or 3-thienyl, 2-, 3- or 4-pyridyl, 2- or 3-furyl, 2-, 3-, 4-, 5- or 8-quinolyl, 4-isoquinolyl, pyrazinyl, 2- or 4-pyrimidinyl, 3-pyrrolyl, 2-imidazolyl, 3-pyridazinyl, 3-isothiazolyl, 3-isoxazolyl, 1-indolyl, 2-indolyl and 2-isoindolinyl, and nonaromatic heterocyclic groups such as 1-, 2- or 3-pyrrolidinyl, 2- or 4-imidazolinyl, 2-, 3- or 4-pyrazolidinyl, piperidino, 2-, 3- or 4-piperidyl, 1- or 2-piperazinyl and morpholino. Among them, for example, a 5- or 6-membered heterocyclic group containing one to three hetero atoms selected from a nitrogen atom, a sulfur atom and an oxygen atom in addition to carbon atoms is preferable, and specifically, 2-thienyl, 3-thienyl, 2-pyridyl, 3-pyridyl, 4-pyridyl, 2-furyl, 3-furyl, pyrazinyl, 2-pyrimidinyl, 3-pyrrolyl, 3-pyridazinyl, 3-isothiazolyl, 3-isoxazolyl, 1-, 2- or 3-pyrrolidinyl, 2- or 4-imidazolinyl, 2-, 3- or 4-pyrazolidinyl, piperidino, 2-, 3-or 4-piperidyl, 1- or 2-piperazinyl, or morpholino is used.

The "$C_{1-6}$ alkyl" represented by $R^b$ includes methyl, ethyl, propyl, isopropyl, butyl, isobutyl, sec-butyl, tert-butyl, pentyl and hexyl.

The "nitrogen-containing heterocyclic ring" which $R^a$ and $R^b$ together with the adjacent nitrogen atom form includes a 5- to 7-membered nitrogen-containing heterocyclic ring which contains at least one nitrogen atom and may contain one to three heteroatoms selected from a nitrogen atom, a sulfur atom and an oxygen atom, in addition to carbon atoms, for example, piperidine, morpholine, thiomorpholine, piperazine and pyrrolidine.

Preferable examples of the "acyl" exemplified as a "substituent" for the "aromatic group" represented by Ar include formyl, carboxy, carbamoyl, optionally halogenated $C_{1-6}$ alkyl-carbonyl, $C_{1-6}$ alkoxy-carbony, (for example, methoxycarbonyl, ethoxycarbonyl, propoxycarbonyl, or tert-butoxycarbonyl), optionally substituted $C_{6-10}$ aryl-carbonyl, optionally substituted $C_{6-10}$ aryloxy-carbonyl, optionally substituted $C_{7-16}$ aralkyloxy-carbonyl, optionally substituted 5- to 6-membered heterocyclic carbonyl, mono-$C_{1-6}$ alkyl-carbamoyl, di-$C_{1-6}$ alkyl-carbamoyl (for example, dimethylcarbamoyl, diethylcarbamoyl, or ethylmethylcarbamoyl), optionally substituted $C_{6-10}$ aryl-carbamoyl, optionally substituted 5- to 6-membered heterocyclic carbamoyl, optionally halogenated $C_{1-6}$ alkylsulfonyl, and optionally substituted $C_{6-10}$ arylsulfonyl.

Among them, the "$C_{6-10}$ aryl-carbonyl" of the "optionally substituted $C_{6-10}$ aryl-carbonyl" includes benzoyl, 1-naphthoyl and 2-naphthoyl. The "$C_{6-10}$ aryloxy-carbonyl" of the "optionally substituted $C_{6-10}$ aryloxy-carbonyl" includes phenoxycarbonyl. The "$C_{7-16}$ aralkyloxy-carbonyl" of the "optionally substituted $C_{7-16}$ aralkyloxycarbonyl" includes benzyloxycarbonyl and phenethyloxycarbonyl. The "5- to 6-membered heterocyclic carbonyl" of the "optionally substituted 5- to 6-membered heterocyclic carbonyl" includes nicotinoyl, isonicotinoyl, 2-thenoyl, 3-thenoyl, 2-furoyl, 3-furoyl, morpholinocarbonyl, piperidinocarbonyl and 1-pyrrolidinylcarbonyl. The "$C_{6-10}$ aryl-carbamoyl" of the "optionally substituted $C_{6-10}$ aryl-carbamoyl" includes phenylcarbamoyl, 1-naphthylcarbamoyl and 2-naphthylcarbamoyl. The "5- to 6-membered heterocyclic carbamoyl" of the "optionally substituted 5- to 6-membered heterocyclic carbamoyl" includes 2-pyridylcarbamoyl, 3-pyridylcarbamoyl, 4-pyridylcarbamoyl, 2-thienylcarbamoyl, and 3-thienylcarbamoyl. The "$C_{6-10}$ arylsulfonyl" of the "optionally substituted $C_{6-10}$ arylsulfonyl" includes benzensulfonyl, 1-naphthalenesulfonyl and 2-naphthalelesulfonyl.

A "substituent" for these "optionally substituted $C_{6-10}$ arylcarbonyl", "optionally substituted $C_{6-10}$ aryloxy-carbonyl", "optionally substituted $C_{7-16}$ aralkyloxy-carbonyl", "optionally substituted 5- to 6-membered heterocyclic carbonyl", "optionally substituted $C_{6-10}$ aryl-carbamoyl", "optionally substituted 5- to 6-membered heterocyclic carbamoyl" and "optionally substituted $C_{6-10}$ arylsulfonyl" includes halogen atoms, $C_{1-3}$ alkylenedioxy, nitro, cyano, optionally hal-

ogenated $C_{1-6}$ alkyl, optionally halogenated $C_{1-6}$ alkoxy, optionally halogenated $C_{1-6}$ alkylthio, hydroxy, amino, mono-$C_{1-6}$ alkylamino, di-$C_{1-6}$ alkylamino, formyl, carboxy, carbamoyl, optionally halogenated $C_{1-6}$ alkyl-carbonyl, $C_{1-6}$ alkoxy-carbonyl, mono-$C_{1-6}$ alkyl-carbamoyl, di-$C_{1-6}$ alkyl-carbamoyl, optionally halogenated $C_{1-6}$ alkylsulfonyl, formylamino, optionally halogenated $C_{1-6}$ alkyl-carboxamido, $C_{1-6}$ alkoxy-carboxamido, $C_{1-6}$ alkylsulfonylamino, $C_{1-6}$ alkyl-carbonyloxy, $C_{1-6}$ alkoxy-carbonyloxy, mono-$C_{1-6}$ alkyl-carbamoyloxy and di-$C_{1-6}$ alkylcarbamoyloxy, and one to five, preferably one to three of these substituents may be used.

The "acylamino" exemplified as a "substituent" for the "optionally substituted aromatic group" represented by Ar mentioned above includes mono- or di-substituted amino with the "acyl" described in detail in the explanation of a "substituent" for the "optionally substituted aromatic group" represented by Ar mentioned above, and preferably, acylamino represented by the following formula: $-NR^c-COR^d$, $-NR^c-COOR^{da}$, $-NR^c-SO_2RR^{da}$, or $-NR^c-CONR^{da}R^{db}$ wherein, $R^c$ is a hydrogen atom or $C_{1-6}$ alkyl, $R^d$ has the same meaning as that of $R^a$ mentioned above, $R^{da}$ has the same meaning as that of $R^{aa}$ mentioned above, and $R^{db}$ has the same meaning as that of $R^b$.

The "$C_{1-6}$ alkyl" represented by $R^c$ and $R^{db}$ includes similar groups to "$C_{1-6}$ alkyl" represented by $R^b$.

The "acylamino" exemplified as a "substituent" for the "optionally substituted aromatic group" represented by Ar includes preferably formylamino, optionally halogenated $C_{1-6}$ alkyl-carboxamido, optionally substituted $C_{6-10}$ aryl-carboxamido (for example, phenylcarboxamido or naphthylcarboxamido), $C_{1-6}$ alkoxy-carboxamido (for example, methoxycarboxamido, ethoxycarboxamido, propoxycarboxamido, or butoxycarboxamido), and $C_{1-6}$ alkylsulfonylamino (for example, methylsulfonylamino or ethylsulfonylamino).

The "acyloxy" exemplified as a "substituent" for the "optionally substituted aromatic group" represented by Ar mentioned above includes mono-substituted oxy with the "acyl" described in detail in the explanation of a "substituent" for the "optionally substituted aromatic group" represented by Ar mentioned above, and preferably, acyloxy represented by the following formula: $-O-COR^e$, $-O-COOR^e$, or $-O-CONHR^e$
wherein, $R^e$ has the same meaning as that of $R^a$ mentioned above.

The "acyloxy" exemplified as a "substituent" for the "optionally substituted aromatic group" represented by Ar includes preferably, $C_{1-6}$ alkyl-carbonyloxy (for example, acetoxy or propanoyloxy), optionally substituted $C_{6-10}$ aryl-carbonyloxy (for example, benzoyloxy, 1-naphthoyloxy, or 2-naphthoyloxy), $C_{1-6}$ alkoxy-carbonyloxy (for example, methoxycarbonyloxy, ethoxycarbonyloxy, propoxycarbonyloxy, or butoxycarbonyloxy), mono-$C_{1-6}$ alkyl-carbamoyloxy (for example, methylcarbamoyloxy or ethylcarbamoyloxy), di-$C_{1-6}$ alkyl-carbamoyloxy (for example, dimethylcarbamoyloxy or diethylcarbamoyloxy), optionally substituted $C_{6-10}$ aryl-carbamoyloxy (for example, phenylcarbamoyloxy or naphthylcarbamoyloxy) and nicotinoyloxy. A "substituent" for these "optionally substituted $C_{6-10}$ aryl-carboxamido", "optionally substituted $C_{6-10}$ aryl-carbonyloxy" and "optionally substituted $C_{6-10}$ aryl-carbamoyloxy" and "preferable example" thereof include similar groups to the "substituent" for the "optionally substituted $C_{6-10}$ arylcarbonyl" mentioned above.

Among the above mention, preferably Ar may represent an optionally substituted ring-assembly aromatic group (in particular, biphenylyl such as 2-, 3- or 4-biphenylyl).

In the compound (I), X is a divalent $C_{1-6}$ aliphatic hydrocarbon group which may contain one or two divalent groups selected from -O-, -S-, -CO-, -SO-, $-SO_2-$ and -COO- and Y is a divalent $C_{1-6}$ aliphatic hydrocarbon group.

Said $C_{1-6}$ aliphatic hydrocarbon group includes $C_{1-6}$ alkylene, $C_{2-6}$ alkenylene and $C_{2-6}$ alkynylene.

Said $C_{1-6}$ alkylene includes $C_{1-3}$ alkylene (for example, $-CH_2-$, $-(CH_2)_2-$, or $-(CH_2)_3-$) which may contain one to three $C_{1-3}$ alkyl, as well as straight-chain $C_{1-6}$ alkylene such as $-CH_2-$, $-(CH_2)_2-$, $-(CH_2)_3-$, $-(CH_2)_4-$, $-(CH_2)_5-$ and $-(CH_2)_6-$.

Said $C_{2-6}$ alkenylene includes $C_{2-3}$ alkenylene (for example, -CH=CH- or $-CH_2-CH=CH-$) which may have one to three $C_{1-3}$ alkyl, as well as straight-chain $C_{2-6}$ alkenylene such as -CH=CH- and $-CH_2-CH=CH-$.

Said $C_{2-6}$ alkynylene includes $C_{2-3}$ alkynylene (for example, $-C\equiv C-$, $-CH_2-C\equiv C-$, $-C\equiv C-CH_2-$, $-C\equiv C-CH_2CH_2-$, or $-CH_2CH_2-C\equiv C-$) which may have one to three $C_{1-3}$ alkyl, as well as straight-chain $C_{2-6}$ alkynylene such as $-C\equiv C-$, $-CH_2-C\equiv C-$, $-C\equiv C-CH_2-$, $-C\equiv C-CH_2CH_2-$, $-CH_2CH_2-C\equiv C-$, $-CH_2-C\equiv C-CH_2-$, $-(CH_2)_2-C\equiv C-CH_2-$, $-(CH_2)_2-C\equiv C-(CH_2)_2-$, and $-(CH_2)_3-C\equiv C-CH_2-$.

Said $C_{1-6}$ aliphatic hydrocarbon group may be preferably a $C_{1-3}$ aliphatic hydrocarbon group such as $C_{1-3}$ alkylene, $C_{2-3}$ alkenylene and $C_{2-6}$ alkynylene.

In particular, preferably, X is $C_{1-3}$ alkylene containing one -O- and Y is $C_{1-3}$ alkylene.

In the compound (I), $R^1$ and $R^2$ may be the same or different and each indicates optionally substituted $C_{1-6}$ alkyl.

The "$C_{1-6}$ alkyl" of the "optionally substituted $C_{1-6}$ alkyl" represented by $R^1$ and $R^2$ includes methyl, ethyl, propyl, isopropyl, butyl, isobutyl, sec-butyl, tert-butyl, pentyl and hexyl, and among them, methyl, ethyl and propyl are preferable.

A "substituent" for the "optionally substituted $C_{1-6}$ alkyl" represented by R' or R" includes halogen atoms (for example, fluorine, chlorine, bromine, or iodine), $C_{1-3}$ alkylenedioxy (for example, methylenedioxy or ethylenedioxy), nitro, cyano, optionally halogenated $C_{1-6}$ alkyl, optionally halogenated $C_{3-6}$ cycloalkyl, optionally halogenated $C_{1-6}$

alkoxy, optionally halogenated $C_{1-6}$ alkylthio, hydroxy, amino, mono-$C_{1-6}$ alkylamino (for example, methylamino, ethylamino, propylamino, isopropylamino or butylamino), di-$C_{1-6}$ alkylamino (for example, dimethylamino, diethylamino, dipropylamino, dibutylamino, or ethylmethylamino), formyl, carboxy, carbamoyl, optionally halogenated $C_{1-6}$ alkyl-carbonyl, $C_{1-6}$ alkoxy-carbonyl (for example, methoxycarbonyl, ethoxycarbonyl, propoxycarbonyl, or tert-butoxycarbonyl), mono-$C_{1-6}$ alkyl-carbamoyl (for example, methylcarbamoyl or ethylcarbamoyl), di-$C_{1-6}$ alkyl-carbamoyl (for example, dimethylcarbamoyl, diethylcarbamoyl or diethylmethylcarbamoyl), optionally halogenated $C_{1-6}$ alkylsulfonyl, formylamino, optionally halogenated $C_{1-6}$ alkyl-carboxamido, $C_{1-6}$ alkoxy-carboxamido (for example, methoxycarboxamido, ethoxycarboxamido, propoxycarboxamido, or butoxycarboxamido), $C_{1-6}$ alkylsulfonylamino (for example, methylsulfonylamino or ethylsulfonylamino), $C_{1-6}$ alkyl-carbonyloxy (for example, acetoxy or propanoyloxy), $C_{1-6}$ alkoxy-carbonyloxy (for example, methoxycarbonyloxy, ethoxycarbonyloxy, propoxycarbonyloxy, or butoxycarbonyloxy), mono-$C_{1-6}$ alkyl-carbamoyloxy (for example, methylcarbamoyloxy or ethylcarbamoyloxy), di-$C_{1-6}$ alkyl-carbamoyloxy (for example, dimethylcarbamoyloxy or diethylcarbamoyloxy) and optionally substituted aromatic groups, and one to five, preferably one to three of these substituents may be used. If the number of substituents is two or more, the individual substituents may be either the same or different from each other.

In the compound (I), ring A indicates a benzene ring which may have an additional substituent. Namely, the ring A may have an additional substituent in addition to the group represented by the formula Ar-X- at the substitutable position. Such a substituent includes halogen atoms (for example, fluorine, chlorine, bromine, or iodine), optionally halogenated $C_{1-6}$ alkyl, optionally halogenated $C_{1-6}$ alkoxy, hydroxy and amino. The "optionally halogenated $C_{1-6}$ alkyl" and "optionally halogenated $C_{1-6}$ alkoxy" include similar groups to the "optionally halogenated $C_{1-6}$ alkyl" and "optionally halogenated $C_{1-6}$ alkoxy" described in detail in the explanation of Ar mentioned above, respectively. A substituent for the ring A includes, preferably, halogen atoms (for example, chlorine) and $C_{1-6}$ alkoxy (for example, methoxy). The ring A may be substituted with one to three of such substituents at the substitutable positions, and if it has two or more substituents, each substituent may be the same or different. In particular, the ring A is preferably substituted with the only substituent represented by the formula Ar-X-.

In the compound (I), ring B indicates a 4- to 8-membered ring which may have an additional substituent.

The 4- to 8-membered ring represented by the ring B includes a 4- to 8-membered homocyclic or heterocyclic ring which may have one double bond in addition to the site fused with the ring A and may have one to three heteroatoms selected from an oxygen atom, a nitrogen atom and a sulfur atom in addition to carbon atoms. A specific example thereof includes a ring represented by the following formula:

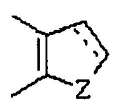

wherein, $\overline{- - -}$ is a single or double bond, and Z is (i) a bond, (ii) $C_{1-4}$alkylene or (iii) $C_{2-4}$ alkenylene. Z is preferably $C_{1-3}$ alkylene and more preferably, ethylene.

Said "4- to 8-membered ring" includes, preferably, a ring represented by the following formula:

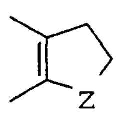

wherein, Z has the same meaning as that mentioned above. Preferably, such a ring is a 6-membered homocyclic or heterocyclic ring which does not contain a double bond other than the site fused with the ring A and may contain one oxygen atom or imino in addition to carbon atoms.

The "substituent" for the "4- to 8-membered ring which may have an additional substituent" represented by the ring B includes oxo, $C_{1-6}$ alkyl (for example, methyl, ethyl, propyl, isopropyl, or butyl) and hydroxy. Such a ring may be substituted with one to three of these substituents at the substitutable positions and if it has two or more substituents, the individual substituents may be either the same or different from each other.

The ring B is preferably a 6-membered homocyclic or heterocyclic ring which does not have a substituent other than the group represented by the following formula:

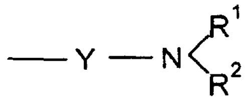

.

The fused ring of the ring A and the ring B includes preferably rings represented by the following formula:

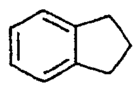 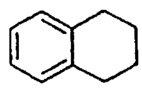 or 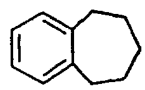

Among them, tetralin is preferable.

The compound (I) includes, in particular, 6-(4-biphenylyl) methoxy-2-[2-(N,N-dimethylamino) ethyl] tetralin (also referred as 6-[(1,1'-biphenyl)-4-ylmethoxy]-1,2,3,4-tetrahydro-N,N'-dimethyl-2-naphtaleneethanamine), 6-(4-biphenylyl)methoxy-2-(N,N-dimethylamino)methyltetralin, 6-(4-biphenylyl)methoxy-2-(N,N-dipropylamino)methyltetralin, 2-(N,N-dimethylamino)methyl-6-(4'-methoxybiphenyl-4-yl)methoxytetralin, 6-(4-biphenylyl)methoxy-2-[2-(N,N-diethylamino)ethyl]tetralin, 2-[2-(N,N-dimethylamino)ethyl]-6-(4'-methylbiphenyl-4-yl)methoxytetralin, 2-[2-(N,N-dimethylamino)ethyl]-6-(4'-methoxybiphenyl-4-yl)methoxytetralin, 6-(2',4'-dimethoxybiphenyl-4-yl)methoxy-2-[2-(N,N-dimethylamino)ethyl]tetralin, 6-[4-(1,3-benzodioxol-5-yl)phenyl]methoxy-2-[2-(N,N-dimethylamino)ethyl]tetralin, and 6-(3',4'-dimethoxybiphenyl-4-yl)methoxy-2-[2-(N,N-dimethylamino)ethyl]tetralin, and optically active forms and hydrates thereof. Among them, preferred are (R)-(+)-6-(4-biphenylyl)methoxy-2-[2-(N,N-dimethylamino)ethyl]tetralin (also referred as (R)-6-[(1,1'-biphenyl)-4-ylmethoxy]-1,2,3,4-tetrahydro-N,N-dimethyl-2-naphtaleneethanamine) hydrochloride monohydrate (compound A), (+)-6-(4-biphenylyl)methoxy-2-[2-(N,N-diethylamino)ethyl]tetralin, (+)-2-[2-(N,N-dimethylamino)ethyl]-6-(4'-methylbiphenyl-4-yl)methoxytetralin, (+)-2-[2-(N,N-dimethylamino)ethyl]-6-(4'-methoxybiphenyl-4-yl)methoxytetralin, (+)-6-(2',4'-dimethoxybiphenyl-4-yl)methoxy-2-[2-(N,N-dimethylamino)ethyl]tetralin, (+)-6-[4-(1,3-benzodioxol-5-yl)phenyl]methoxy-2-[2-(N,N-dimethylamino)ethyl]tetralin, and (+)-6-(3',4'-dimethoxybiphenyl-4-yl)methoxy-2-[2-(N,N-dimethylamino)ethyl]tetralin.

The compound (I) can be produced according to the method disclosed in JP-A 11-80098 (WO98/38156) or WO01/74756. Alternatively, if an amide bond and an ether bond are present in the same molecule, the compound may be produced by an improved method wherein only the ether bond is selectively broken in the presence of methanesulfonic acid and methionine and then alkylation reaction is undergone to reduce the amide moiety.

Another compound having an inhibitory activity on β-secretase which may be used in the present invention is, for example, OM99-2 (Science, 290, 150-153; J. Am. Chem. Soc., 2000, 122, 3522-3523) represented by the following formula:

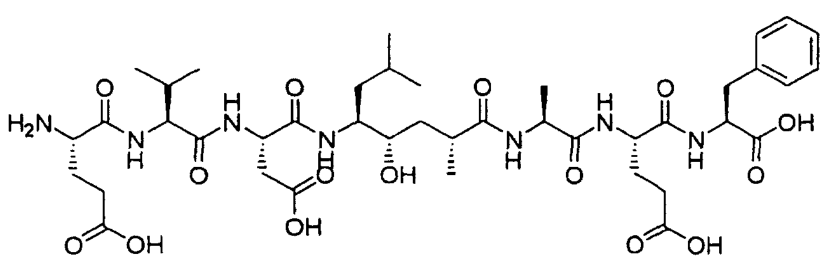

.

Compounds having an inhibitory activity on γ-secretase which may be used in the present invention include L-685458 represented by the following formula:

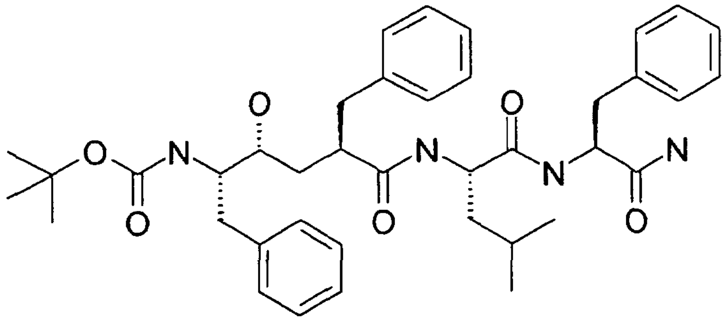

,

LY-374973 (Investigation Drugs, db, 2001.1) represented by the following formula:

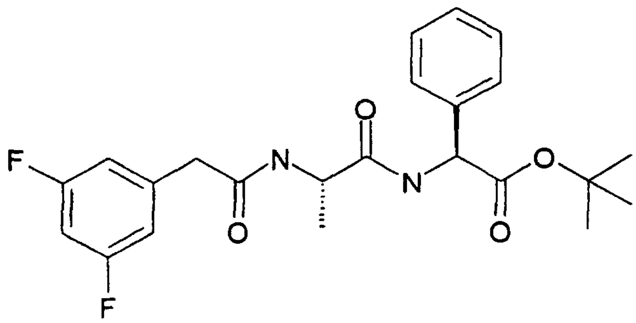

and RF-978 represented by the following formula:

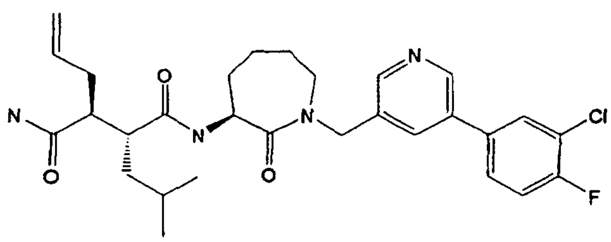

Compounds having an inhibitory activity on aggregation of β protein which may be used in the present invention include PTI-00703, ALZHEMED (NC-531), PPI-368 represented by the following formula:

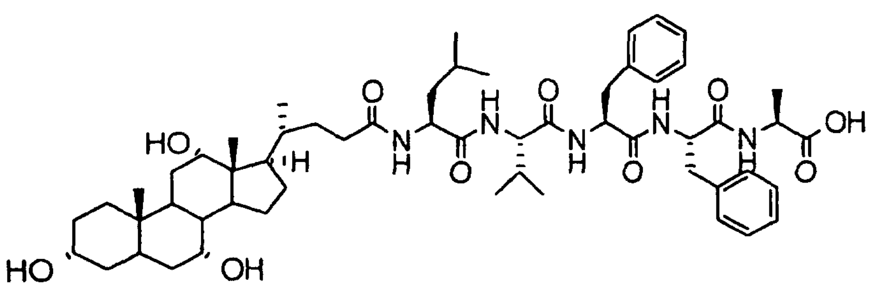

and PPI-558(WO98/08868), SKF-74652 represented by the following formula:

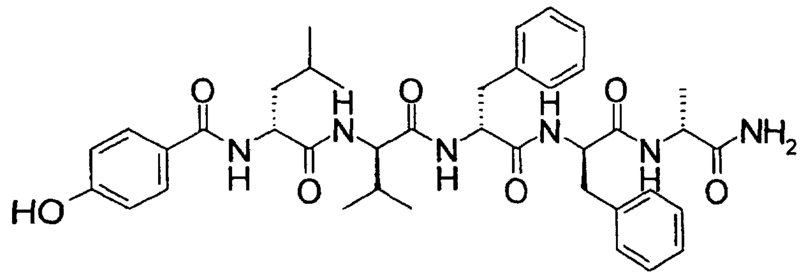

.

Salts of compounds having an inhibitory effect on production, secretion, aggregation and/or accumulation of β-amyloid protein include salts with inorganic bases, ammonium salts, salts with organic bases, salts with inorganic acids, salts with organic acids, and salts with basic or acidic amino acids.

Preferable examples of salts with inorganic bases include alkali metal salts such as a sodium salt and a potassium salt; alkali earth metal salts such as a calcium salt, a magnesium salt and a barium salt; and a aluminum salt. Preferable examples of salts with organic bases include salts with trimethylamine, triethylamine, pyridine, picoline, ethanolamine, diethanolamine, triethanolamine, dicyclohexylamine or N,N-dibenzylethylenediamine. Preferable examples of salts with inorganic acids include salts with hydrochloric acid, hydrobromic acid, nitric acid, sulfuric acid or phosphoric acid. Preferable examples of salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, or p-toluenesulfonic acid. Preferable examples of salts with basic amino acids include salts with arginine, lysine or ornithine and preferable examples of salts with acidic amino acids include salts with aspartic acid or glutamic acid.

Among these salts, pharmacologically acceptable salts are preferred. For example, if the compound having an inhibitory effect on production, secretion, aggregation and/or accumulation of β-amyloid protein contains an acidic functional group, an inorganic salt such as an alkali metal salt (for example, a sodium salt or a potassium salt) or an alkali earth metal salt (for example, a calcium salt, a magnesium salt, or a barium salt) or an ammonium salt is used. If the compound contains a basic functional group, an inorganic salt such as hydrochloride, sulfate, phosphate or hydrobromate or an organic salt such as acetate, maleate, fumarate, succinate, methanesulfonate, p-toluenesulfonate, citrate or tartrate is used. In the case of the compound (I), among them, hydrochloride, citrate and fumarate are preferable.

Moreover, the compound having an inhibitory effect on production, secretion, aggregation and/or accumulation of β-amyloid protein may be anhydrous or hydrous. If being hydrous, the compound may contain one to three $H_2O$ molecules.

The compound having an inhibitory effect on production, secretion, aggregation and/or accumulation of β-amyloid protein which may be used in the present invention, for example, the compound (I) may be in the stereoisomeric form depending on the kind of substituents which the compound has, and the single stereoisomer or a mixture of the stereoisomers may be used in the present invention.

Moreover, if the compound (I) exists as a configurational isomer, diastereomer, conformer or the like, if desired, the isomers may be separated according to a known method for separating and purifying them. In addition, if the compound (I) is racemic, it may be separated into d-form and 1-form using a standard optical resolution technique. In the present invention, though these separated isomers or a mixture of these isomers may be used, use of optically active substances is more suitable in many cases.

The compound having an inhibitory effect on production, secretion, aggregation and/or accumulation of β-amyloid protein may be a prodrug thereof. Said prodrug means a compound which is converted into a compound having an inhibitory effect on production, secretion, aggregation and/or accumulation of β-amyloid protein by a reaction with an enzyme, gastric acid or the like under the in vivo physiological condition, namely (1) a compound which is converted into the desired compound by enzymatic oxidation, reduction, hydrolysis or the like or (2) a compound which is converted into the desired compound by hydrolysis or the like with gastric acid. The prodrug of the compound having an inhibitory effect on production, secretion, aggregation and/or accumulation of β-amyloid protein includes a compound whose hydroxyl group is acylated, alkylated, phosphorylated or borated or a salt thereof (for example, a compound whose hydroxyl group is acetylated, palmitoylated, propanoylated, pivaloylated, succinylated, fumarylated, alanylated or dimethylaminomethylcarbonylated or a salt thereof) and a compound whose carboxyl group is esterified or amidated (for example, compound whose carboxyl group is ethylesterified, phenylesterified, carboxyoxymethylesterified, dimethylaminomethylesterified, pivaloyloxymethylesterified, ethoxycarbonyloxyethylesterified, phthalidylesterified, (5-methyl-2-oxo-1,3-dioxolen-4-yl)methylestrified, cyclohexyloxycarbonylethylesterified or methylamidated, or a salt thereof). These prodrugs can be produced according to any method known per se or its modification.

Alternatively, prodrugs of the compounds having an inhibitory effect on production, secretion, aggregation and/or accumulation of β-amyloid protein may be converted into the compounds having an inhibitory effect on production, secretion, aggregation and/or accumulation of β-amyloid protein under the physiological condition described in "Development of Medical Drugs" (Hirokawashoten, vol.7 Molecular design, pp.163-198, 1990).

The compounds having an inhibitory effect on production, secretion, aggregation and/or accumulation of β-amyloid protein may be labeled with an isotope (for example, $^{2}H$, $^{3}H$, $^{14}C$, $^{35}S$, or $^{125}I$) or the like.

The above-mentioned compounds having an inhibitory effect on production, secretion, aggregation and/or accumulation of β-amyloid protein (including a salt thereof) or a prodrug thereof (hereafter, simply referred as the β-amyloid protein-inhibiting compound) is useful in treating mild cognitive impairment because of its inhibitory effect on production, secretion, aggregation and/or accumulation of β-amyloid protein.

The β-amyloid protein inhibiting compounds may be used in treating mild cognitive impairment in combination with, for example, therapeutic drugs of Alzheimer's disease (for example, cholinesterase inhibitors such as donepezil, rivastigmine, galantamine and TAK-147, or brain function activators such as idebenone, memantine and vinpocetine), antiparkinson drugs (for example, L-dopa, deprenyl, carbidopa+levodopa, pergolide, ropinirol, cabergoline, pramipexole, entacapone, or lazabemide), amyotrophic lateral sclerosis therapeutic drugs (for example, riluzole, mecasermin, or gabapentin), neurotrophic factors, antidepressants (for example, fluoxetine, sertraline, paroxetine, venlafaxine, nefazodone, reboxetine, imipramine hydrochloride, or duloxetine), schizophrenia therapeutic drugs (for example, olanzapine, risperidone, questiapine, or iloperidone), antianxiety drugs (for example, alprazolam, bromazepam, chlordiazepoxide, diazepam, etizolam, flutoprazepam, or lorazepam), sleeping drugs (for example, brotizolam, estazolam, flurazepam, nitrazepam, or triazolam), antihyperlipemic drugs (for example, simvastatin, fluvastatin, pravastatin, or atrovastatin), antihypertensive drugs (for example, captopril, delapril, enalapril, nifedipine, nicardipine, amlodipine, alprenolol, propranolol, metroprolol, losartan, valsartan, or candesartan), antiplatelet drugs (for example, ticlopidine, heparin, urokinase, alteplase, tisokinase, nasaruplase or cilostazol), antioxidants (for example, linolenic acid, ascorbic acid, icosapentaenoic acid, docosahexaenoic acid, or tocopherol), vitamins (for example, tocopherol or ascorbic acid), sex hormones (for example, estrogen, estrone, or estradiol), antiinflammatory drugs (for example, predonisolone, betamethasone, or dexamethasone), nonsteroidal antiinflammatory drugs (for example, indomethacin, ibuprofen, acetylsalicylic acid, diclofenac, naproxen, or piroxicam), COX-2 inhibitors (for example, celecoxib or rofecoxib), drugs for improving brain circulation and metabolism (for example, nicergoline, ibudilast, or ifenprodil), anticonvulsants (for example, carbamazepin, valproic acid, clonazepam, vigabatrin, lamotrigine or gabapentin) or pnarmacologically acceptable salts thereof.

The β-amyloid protein inhibiting compound used in the present invention, in particular the compound (I), in combination with the above-mentioned drug, can be also effectively used for prevention or treatment of not only mild cognitive impairment, but also neurodegenerative diseases (for example, senile dementia, Alzheimer's disease, Down's disease, Parkinson's disease, Creutzfeldt-Jakob disease, amyotrophic lateral sclerosis, or diabetic neuropathy), cerebrovascular disorders (for example, brain circulation disorder associated with cerebral infarction, intracerebral hemorrhage or cerebral arteriosclerosis), nervous disorders associated with head injury/spinal cord injury, sequelae of encephalitis or cerebral palsy, memory deficit (for example, senile dementia or amnesia) or mental diseases (for example, depression, panic disorder or schizophrenia).

The β-amyloid protein inhibiting compound, in particular compound (I), or a salt or prodrug thereof is low toxic and may be efficiently delivered into the brain.

Accordingly, the β-amyloid protein inhibiting compound including the compound (I) is useful as a safe therapeutic agent for mild cognitive impairment in mammals (for example, rats, mice, guinea pigs, rabbits, sheeps, horses, pigs, cattle, monkeys, or human beings).

The therapeutic agent for mild cognitive impairment of the present invention can be produced by formulation of the above mentioned β-amyloid protein inhibiting compound according to any method known per se. The β-amyloid protein inhibiting compound may be administered safely and orally or parenterally (for example, locally, rectally, or intravenously) as it is or as a pharmaceutical composition, for example, a tablet (including a sugar-coated tablet and a film-coated tablet), a granule, a capsule (including a soft capsule), liquid, injection, a suppository, or a sustained-release agent, which is prepared by mixing with an appropriate quantity of a pharmacologically acceptable carrier in the formulation process.

The content of the β-amyloid protein inhibiting compound in the therapeutic agent for mild cognitive impairment of the present invention can be selected appropriately depending on the efficacy of the compound and the like. For example, the content of the compound (I) is usually about 0.1 to 100 % by weight of the total agent. The dosage of the agent of the present invention depends on a subject to be administered, an administration route, disease to be treated, and the like. For example, when the agent of the present invention is orally administered to an adult patient (about 60 kg) with Alzheimer's disease, the daily dose is about 0.01 to 500 mg, preferably about 0.1 to 100 mg, more preferably

1 to 100 mg based on the active component (compound (I)), which may be administered in one to several portions per day.

A pharmacologically acceptable carrier used in production of the therapeutic agent for mild cognitive impairment of the present invention includes various organic or inorganic carriers which are conventionally used as formulation materials, for example, excipients, lubricants, binders and disintegrants for solid preparations; and solvents, solubilizing agents, suspending agents, isotonizing agents, buffers or soothing agents for liquid preparations. In addition, additives may be used if necessary, including antiseptics, antioxidants, coloring agents, sweetening agents, absorbing agents and wetting agents.

Excipients include lactose, white sugar, D-mannitol, starch, cornstarch, crystalline cellulose and light anhydrous silicic acid.

Lubricants include magnesium stearate, calcium stearate, talc and colloidal silica.

Binders include crystalline cellulose, white sugar, D-mannitol, dextrin, hydroxypropyl cellulose, hydroxypropylmethyl cellulose, polyvinylpyrrolidone, starch, sucrose, gelatin, methyl cellulose and carboxymethyl cellulose sodium.

Disintegrants include starch, carboxymethyl cellulose, carboxymethyl cellulose calcium, croscarmellose sodium, carboxymethyl starch sodium and L-hydroxypropyl cellulose.

Solvents include water for injection, alcohol, propylene glycol, macrogol, sesame oil and corn oil.

Solubilizing agents include polyethylene glycol, propylene glycol, D-mannitol, benzyl benzoate, ethanol, trisaminomethane, cholesterol, triethanolamine, sodium carbonate and sodium citrate.

Suspending agents include surfactants such as stearyltriethanolamine, sodium lauryl sulfate, laurylamino propionic acid, lecithin, benzalkonium chloride, benzethonium chloride and glyceryl monostearate; and hydrophilic polymers such as polyvinyl alcohol, polyvinylpyrrolidone, carboxymethyl cellulose sodium, methyl cellulose, hydroxymethyl cellulose, hydroxyethyl cellulose and hydroxypropyl cellulose.

Isotonizing agents include glucose, D-sorbitol, sodium chloride, glycerin and D-mannitol.

Buffers include buffer solutions of phosphate, acetate, carbonate and citrate.

Soothing agents include benzyl alcohol.

Antiseptics include p-hydroxybenzoic esters, chlorobutanol, benzyl alcohol, phenethyl alcohol, dehydroacetic acid and sorbic acid.

Antioxidants include sulfite salt, ascorbic acid and sodium ascorbate.

For stability in the presence of light or the like, the agent of the present invention may be also coated with a light-blocking coating according to a conventional method. As the coating base, for example, hydroxypropylmethyl cellulose, ethyl cellulose, hydroxymethyl cellulose or the like may be used. To the base, titanium dioxide, iron sesquioxide or the like may be added. As a plasticizer for a film, for example, polyethylene glycol may be further added. In the case of the agent comprising the compound (I), copolyvidone is suitably used as a plasticizer.

## Examples

Heireinbelow, with reference to Referential Examples, Examples and Experimental Examples concerning the compound (I), the present invention is described in detail but is not limited to the compound and applicable to other compounds.

### Referential Example 1

Synthesis of (R)-(+)-6-(4-biphenylyl)methoxy-2-[2-(N,N-dimethylamino)ethyl]tetralin hydrochloride monohydrate

(compound A)

695 g of (+)-N,N-dimethyl-(6-(4-biphenylyl)methoxy-2-tetralin)acetamide (obtained in accordance with the method disclosed in JP-A 11-310561) was suspended in 3475 mL of a toluene solution. To the suspension, 562 g of sodium dihydro-bis(2-methoxyethoxy)aluminate (a 70% solution in toluene) was added dropwise at an internal temperature of 20°C or below under a nitrogen atmosphere. After stirring at room temperature for 1.5 hours, 695 mL of an aqueous 4N sodium hydroxide solution was added dropwise at 20°C or below. The mixture was stirred at room temperature for 30 minutes and then separated. An organic layer was washed twice with 695 mL of an aqueous 1N sodium hydroxide solution and twice with 1390 mL of water. To the organic layer added was 348 mL of toluene, and the mixture was heated up to 60°C and 175 mL of concentrated hydrochloric acid (content: 36%) was added dropwise thereto. After stirring for an hour under ice-cooling, a precipitated crystal was filtered and washed with 695 mL of toluene and 1390 mL of an aqueous 50% methanol solution. The crystal was dried under reduced pressure at 40°C to obtain 723 g (yield: 94.4%) of the titled compound as a pale yellow crystal.

$^1$H-NMR (300MHz, DMSO-$d_6$) δ: 1.32-1.40(1H, m), 1.62-1.74(3H, m), 1.82-1.90(1H, m), 2.28-2.38(1H, m), 2.74(6H, s), 2.76-2.82(3H, br), 3.08-3.16(2H, m), 5.09(2H, s), 6.72-6.80(2H, m), 6.96(1H, d, J=8.0Hz), 7.32-7.38(1H, m), 7.44-7.54(4H, m), 7.64-7.72(4H, m), 10.4(1H, br).

Referential Example 2

Purification of (R)-(+)-6-(4-biphenylyl)methoxy-2-[2-(N,N-dimethylamino)ethyl]tetralin hydrochloride monohydrate

479.8 g of (R)-(+)-6-(4-biphenylyl)methoxy-2-[2-(N,N-dimethylamino)ethyl]tetralin hydrochloride monohydrate obtained in the Referential Example 1 was dissolved in a mixture of 3186 mL of tetrahydrofuran and 864 mL of water at 60°C. To the solution 24 g of activated carbon was added and the mixture was stirred at 60°C for 30 minutes. The activated carbon was filtered out and washed with a mixture of 336 mL of tetrahydrofuran and 216 mL of water. The filtrate was heated up to 60°C and 2688 mL of tetrahydrofuran was added dropwise thereto while being stirring. After the mixture was cooled to room temperature and further stirred at 5 to 10°C for 2 hours, a precipitated crystal was separated by a centrifuge method. The separated crystal was washed with a mixture of 216 mL of tetrahydrofuran and 744 mL of water to obtain the pure titled compound (390.5 g, 85%).

Example 1

A therapeutic agent for mild cognitive impairment of the present invention was produced in accordance with the formulation shown in Table 1.

2.3 g of compound A, 222.2 g of lactose and 50 g of cornstarch were mixed homogeneously in a fluidized-bed granulation dryer. In the dryer, the mixture was sprayed with an aqueous solution containing 9 g of hydroxypropyl cellulose, granulated and then dried. The resulting granules were crushed with a 1.5 mmϕ punching screen of a power mill to obtain sized powder. To 226.8 g of the sized powder, 12 g of croscarmellose sodium and 1.2 g of magnesium stearate were added and mixed to obtain granules for tableting. The granules were compressed into plain tablets (one tablet weight: 150 mg) with a 7.5 mmϕ mallet in a tableting machine. The resulting plain tablets were sprayed with a solution of hydroxypropylmethyl cellulose 2910 and copolyvidone in which titanium dioxide and iron sesquioxide were dispersed in a film coating machine to obtain about 1500 film-coated tablets which contain 1.15 mg of compound A per one tablet as shown in Table 1. Similarly, a 5 mg tablet and a 50 mg tablet were produced.

Table 1

| Formulation | | | |
|---|---|---|---|
| Composition | Contents (mg) | | |
| | 1 mg tablet | 5 mg tablet | 50 mg tablet |
| Compound A | 1.15 | 5.7 | 57.0 |
| Lactose | 111.1 | 116.5 | 55.2 |
| Cornstarch | 25.0 | 25.0 | 25.0 |
| hydroxypropyl cellulose | 4.5 | 4.5 | 4.5 |
| Croscarmellose sodium | 7.5 | 7.5 | 7.5 |
| Magnesium stearate | 0.75 | 0.75 | 0.75 |
| Bare tablet | 150.0 | 150.0 | 150.0 |
| Hydroxypropylmethyl cellulose 2910 | 4.464 | 4.464 | 4.464 |
| Copolyvidone | 0.9 | 0.9 | 0.9 |
| Titanium dioxide | 0.6 | 0.6 | 0.6 |
| Iron sesquioxide | 0.036 | 0.036 | 0.036 |
| Total | 156.0 | 156.0 | 156.0 |

Experimental Example 1

An ameliorative effect of the agent on learning deficit in old rats

1) Method

Fisher344 rats (27-month old, male) were previously subjected to four trials of a water maze test using a pool with a diameter of 90 cm and a platform on the surface of water in the pool. The rats with remarkable disability to swim were excluded and the rest were divided into several groups. The compound A (0.3 or 1.0 mg/kg) was orally administered to the rats once a day for 14 days. On the 10th day, the rats were subjected to four trials of a water maze test using a platform on the surface of water in a pool and confirmed again that they had no difficulty in swimming ability. Starting from the next day, the rats were subjected four times a day for 3 days to a water maze test using a pool with a diameter of 120 cm and a platform submerged below the surface of water in the pool. In this test, the time required for the rats to arrive at the platform was measured. Two trials were expressed as one block. During the behavioral pharmacological test period, the drug was administered after the test.

2) Results

The results are shown in Fig. 1. In the test using a small pool, it was confirmed that the rats had no problem associated with visual power and physical ability. In the water maze test using a large pool, a remarkable difference was observed between the group of younger rats and the group of older rats (control group) (##$P<0.01$). The time required for the older group to arrive at a platform under water was always longer than that of the younger group. In the compound A treatment group, the time required to arrive at the platform was significantly improved. As compared between the groups in the final training block, the swimming time for escape was significantly shortened by the administration of the compound A, suggesting that the compound A improved the learning deficit of old rats.

Experimental Example 2

An effect on increase in insoluble Aβ40/42 and amyloid plaque formation in the brains of transgenic mice with Swedish APP mutation

1) Method

Transgenic mice with Swedish amyloid precursor protein (APP) mutation (7- to 8-month old) were given solid feed containing the compound A (56 ppm) (equivalent to a dosage of about 7 mg/kg/day) or control feed for six months. After six months from the administration, one cerebral hemisphere was used for brain histological examination and the other hemisphere was used for determining the quantity of Aβ40/42. Cerebral cortex was used for measuring the quantity of Aβ. The accumulation type of insoluble Aβ40/42 protein were extracted by homogenization of the cerebral cortex in 25 times amount of Tris buffer (containing a protease inhibitor) followed by centrifugation to remove the supernatant. Then, 19 times amount of 70% formic acid was added to the insoluble precipitation and homogenized to solubilize it. After centrifugation, the supernatant was diluted to 1/20 with 1M Tris solution for neutralization and the diluted supernatant was used for determining the quantity of Aβ40/42 contained therein. Using enzyme-linked immunosorbent assay (ELISA), the quantity of accumulation-type Aβ40/42 was measured.

For histopathological search, two sections containing dorsal hippocampus were prepared from each mouse and then immunostained for Aβ40/42 with BAN50 to measure the number and area of amyloid plaques. A vehicle (control) group consisted of 13 animals and a compound A treatment group consisted of 16 animals. Nine animals of 8-month old at the initiation of drug administration were used.

2) Results

The quantities of insoluble Aβ40 and 42 proteins in the case that a vehicle or the compound A was administered for six months are shown in Fig. 2. In the vehicle group, the quantities of Aβ40 and Aβ42 in 13-14 month old transgenic mice with Swedish APP mutation were about 35 times larger and about 25 times larger than those in the 8-month old mice, respectively. In contrast to the vehicle group, the quantities of Aβ40 and Aβ42 were significantly reduced by about 30% in the compound A treatment group. During the drug administration period, there was no change in the general condition of the mice including weight loss.

The number and area of amyloid plaques are shown in Fig. 3. The administration of the compound A reduced the number and area of amyloid plaques to about 40 to 35% of those in the vehicle group respectively. It is generally

believed that in patients with mild cognitive impairment, amyloid plaques formation has been started already and their mild cognitive impairment would change into Alzheimer's disease when the number and area of the amyloid plaques exceed the given thresholds. In this context, the results of our tests demonstrated that the compound A inhibited β-secretase and thereby inhibited production, secretion, aggregation and/or accumulation of amyloid protein. This suggests that the compound A can suppress the progression of mild memory impairment to Alzheimer's disease.

Industrial Applicability

According to the present invention, effective agents for treating mild memory impairment can be provided and thereby the progression of mild memory impairment to Alzheimer's disease can be suppressed.

## Claims

1. A agent for preventing or treating mild cognitive impairment which comprises a compound having an inhibitory effect on production, secretion, aggregation and/or accumulation of β-amyloid protein or a prodrug thereof.

2. The agent according to claim 1 wherein the compound is a compound having an inhibitory effect on β-secretase.

3. The agent according to claim 1 wherein the compound is a compound represented by the formula:

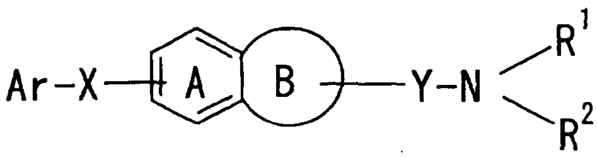

wherein, Ar is an optionally substituted aromatic group, X is a divalent $C_{1-6}$ aliphatic hydrocarbon group which may contain one or two divalent groups selected from -O-, -S-, -CO-, -SO-, -$SO_2$- and -COO-, Y is a divalent $C_{1-6}$ aliphatic hydrocarbon group, $R^1$ and $R^2$ may be the same or different and each is a hydrogen atom or optionally substituted $C_{1-6}$ alkyl, ring A is a benzene ring which may have an additional substituent, and ring B is a 4- to 8-membered ring which may have an additional substituent, or a salt or hydrate thereof.

4. The agent according to claim 3 wherein the compound is selected from 6-(4-biphenylyl)methoxy-2-[2-(N,N-dimethylamino)ethyl]tetralin, 6-(4-biphenylyl)methoxy-2-(N,N-dimethylamino)methyltetralin, 6-(4-biphenylyl)methoxy-2-(N,N-dipropylamino)methyltetralin, 2-(N,N-dimethylamino)methyl-6-(4'-methoxybiphenyl-4-yl)methoxytetralin, 6-(4-biphenylyl)methoxy-2-[2-(N,N-diethylamino)ethyl]tetralin, 2-[2-(N,N-dimethylamino)ethyl]-6-(4'-methylbiphenyl-4-yl)methoxytetralin, 2-[2-(N,N-dimethylamino)ethyl]-6-(4'-methoxybiphenyl-4-yl)methoxytetralin, 6-(2',4'-dimethoxybiphenyl-4-yl)methoxy-2-[2-(N,N-dimethylamino)ethyl]tetralin, 6- [4-(1,3-benzodioxol-5-yl)phenyl]methoxy-2-[2-(N, N-dimethylamino)ethyl]tetralin, 6-(3',4'-dimethoxybiphenyl-4-yl)methoxy-2-[2-(N,N-dimethylamino) ethyl]tetralin, and optical active forms, salts and hydrates thereof.

5. The agent according to claim 2 wherein the compound having an inhibitory effect on β-secretase is OM99-2.

6. The agent according to claim 1 wherein the compound is a compound having an inhibitory effect on γ-secretase.

7. The agent according to claim 1 wherein the compound is a compound having an inhibitory effect on aggregation of β-amyloid protein.

8. The agent according to claim 7 wherein the compound having an inhibitory effect on aggregation of β-amyloid protein is PTI-00703, ALZHEMED (NC-531), PPI-368, PPI-558 or SKF-74652.

9. An agent for inhibiting the progression of mild cognitive impairment to Alzheimer's disease, which comprises a compound having an inhibitory effect on production, secretion, aggregation and/or accumulation of β-amyloid protein or a prodrug thereof.

10. A method for preventing or treating mild cognitive impairment, which comprises administering to a mammal an

effective amount of a compound having an inhibitory effect on production, secretion, aggregation and/or accumulation of β-amyloid protein or a prodrug thereof.

**11.** A method for inhibiting the progression of mild cognitive impairment to Alzheimer's disease, which comprises administering to a mammal an effective amount of a compound having an inhibitory effect on production, secretion, aggregation and/or accumulation of β-amyloid protein or a prodrug thereof.

**12.** Use of a compound having an inhibitory effect on production, secretion, aggregation and/or accumulation of β-amyloid protein or a prodrug thereof, for the manufacture of an agent for preventing or treating mild cognitive impairment.

**13.** Use of a compound having an inhibitory effect on production, secretion, aggregation and/or accumulation of β-amyloid protein or a prodrug thereof, for the manufacture of an agent for inhibiting the progression of mild cognitive impairment to Alzheimer's disease.

Fig. 1

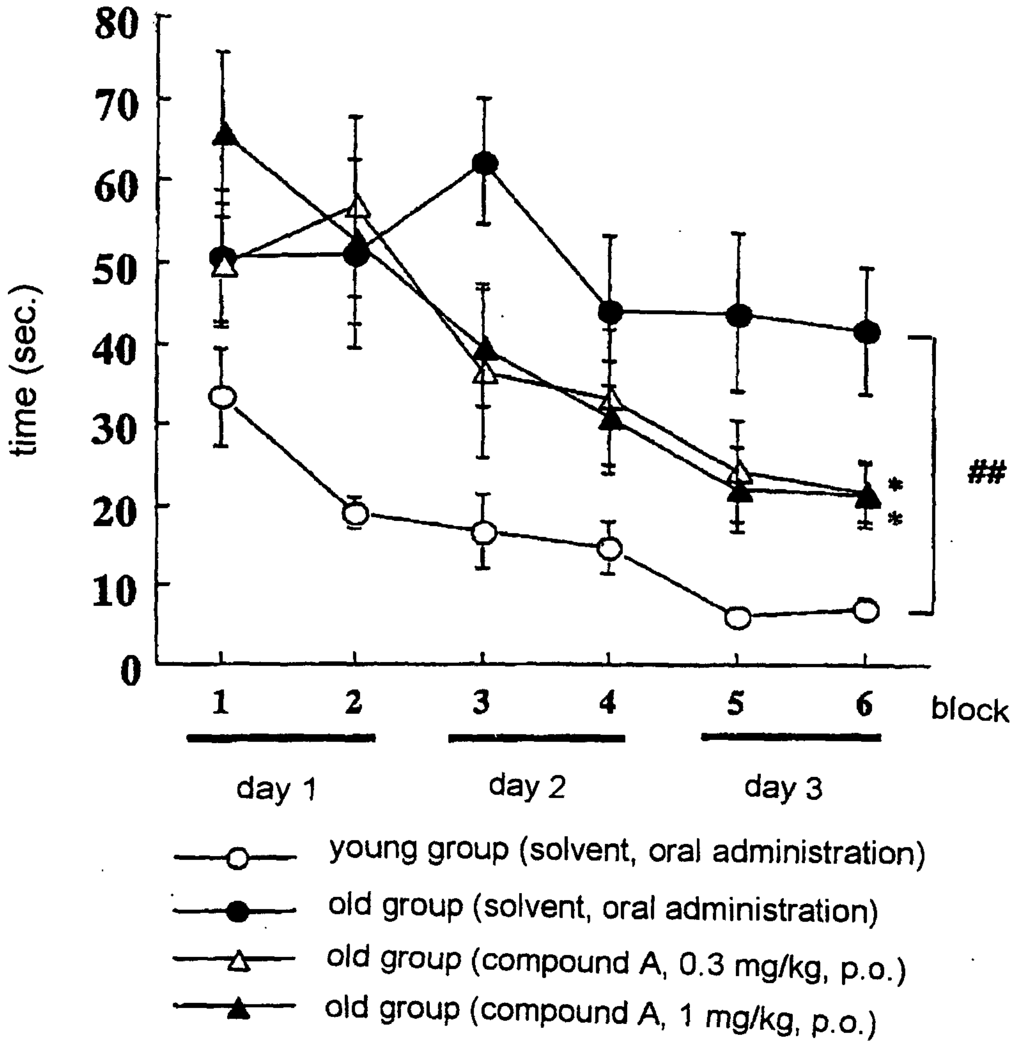
80
70
60
50
40
30
20
10
0
time (sec.)
1
2
3
4
5
6
block
day 1
day 2
day 3
*
*
##
young group (solvent, oral administration)
old group (solvent, oral administration)
old group (compound A, 0.3 mg/kg, p.o.)
old group (compound A, 1 mg/kg, p.o.)

Fig. 2

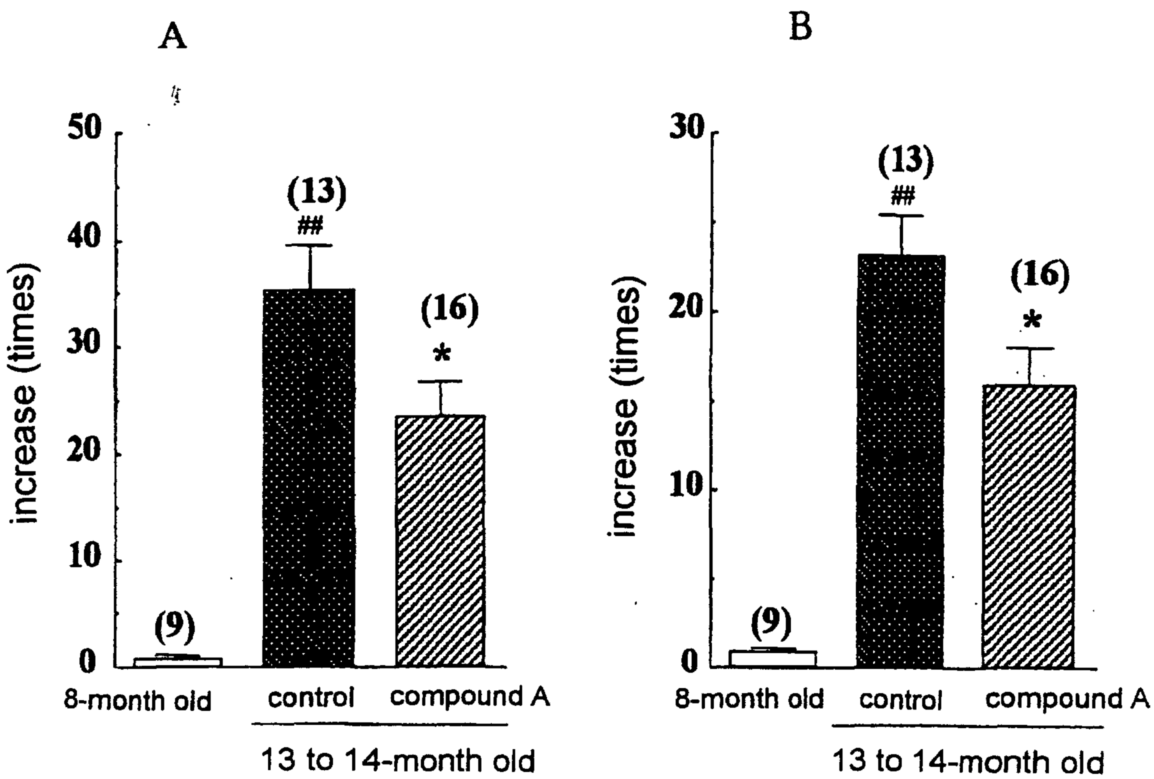
A
50
40
30
20
10
0
increase (times)
(13)
##
(16)
*
(9)
8-month old
control
compound A
13 to 14-month old
B
30
20
10
0
increase (times)
(13)
##
(16)
*
(9)
8-month old
control
compound A
13 to 14-month old

Fig. 3

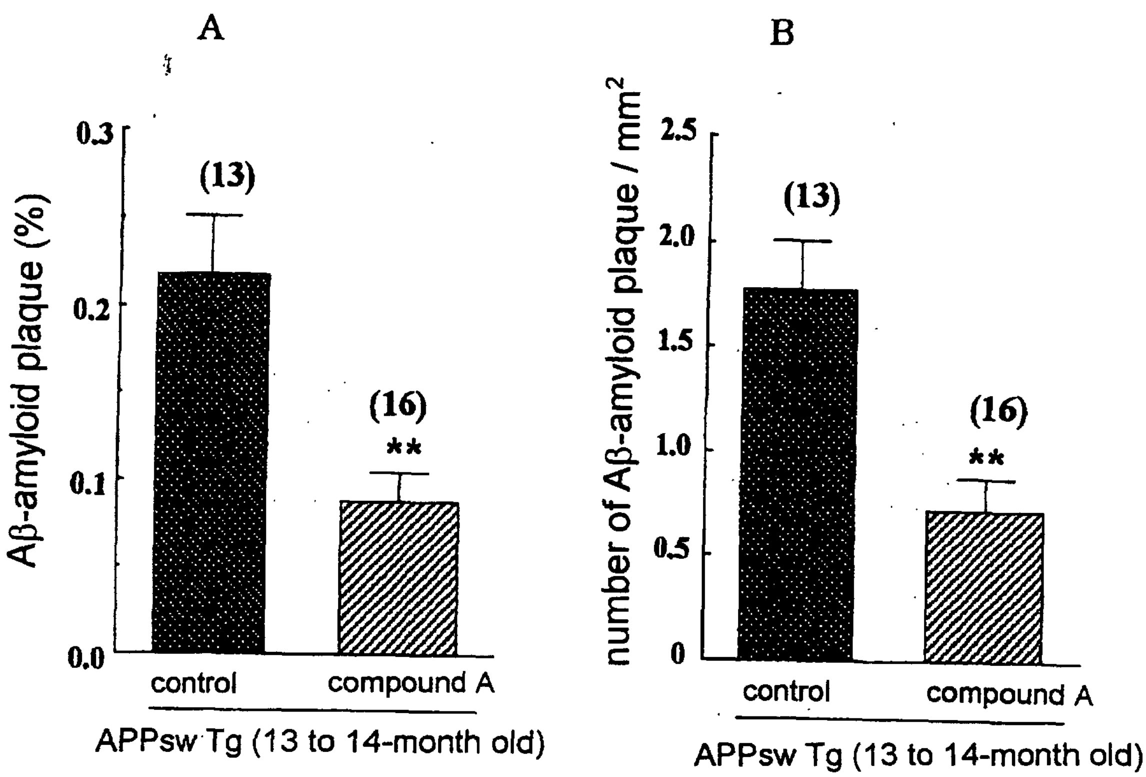
A
B
0.3
0.2
0.1
0.0
Aβ-amyloid plaque (%)
(13)
(16)
**
control
compound A
APPsw Tg (13 to 14-month old)
2.5
2.0
1.5
1.0
0.5
0
number of Aβ-amyloid plaque / mm2
(13)
(16)
**
control
compound A
APPsw Tg (13 to 14-month old)

## INTERNATIONAL SEARCH REPORT

International application No.
PCT/JP02/13478

A. CLASSIFICATION OF SUBJECT MATTER
Int.Cl[7] A61K45/00, 31/135, 38/05, 38/06, 38/08, A61P25/28, 43/00

According to International Patent Classification (IPC) or to both national classification and IPC

B. FIELDS SEARCHED

Minimum documentation searched (classification system followed by classification symbols)
Int.Cl[7] A61K45/00, 31/135, 38/05, 38/06, 38/08, A61P25/28, 43/00

Documentation searched other than minimum documentation to the extent that such documents are included in the fields searched

Electronic data base consulted during the international search (name of data base and, where practicable, search terms used)
CAPLUS(STN), MEDLINE(STN), EMBASE(STN), BIOSIS(STN)

C. DOCUMENTS CONSIDERED TO BE RELEVANT

| Category* | Citation of document, with indication, where appropriate, of the relevant passages | Relevant to claim No. |
|---|---|---|
| X<br>Y | THAL, L.J., Trials to slow progression and prevent disease onset., Journal of Neural Transmission. Supplementum, 2000, Vol.59, pp.243-9, particularly, Summary | 1,7,9,12,13<br>2-6,8 |
| X<br>Y | Chem.abstr., 2000, Vol.135(Columbus, OH, USA), the abstract No.2294, Jin, Liying et al., The effect on serum amyloid β protein of patients withmild cognitive impairment after semiconductorlaser therapy, Quigdao Daxue Yixueyuan Xuebao, 2000, 36(3), 175-176(Chin) | 1,7,9,12,13<br>2-6,8 |
| Y | WO 98/38156 A1 (Takeda Chemical Industries, Ltd.), 03 September, 1998 (03.09.98), Full text<br>& EP 971878 A1 & US 6310107 B1<br>& US 2002/032189 A1 & JP 11-080098 A2<br>& AU 9861166 A1 | 3,4 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 April, 2003 (08.04.03) | 30 April, 2003 (30.04.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/13478

C (Continuation). DOCUMENTS CONSIDERED TO BE RELEVANT

| Category* | Citation of document, with indication, where appropriate, of the relevant passages | Relevant to claim No. |
|---|---|---|
| Y | GHOSH, Arun K. et al., Design of potent inhibitors for human brain memapsin 2(β-secretase), Journal of the American Chemical Society, 2000, Vol.122, No.14, pages 3522 to 3523, particularly, abstract | 5 |
| Y | BEHER, Dirk et al., Pharmacological knock-down of the presenilin 1 heterodimer by a novel γ-secretase inhibitor. Implications for presenilin biology, Journal of Biological Chemistry, Nov.2001, Vol.276, No.48, pages 45394 to 45402, particularly, abstract; Fig. 1 | 6 |
| Y | FINDEIS, Mark A. et al., Modified-Peptide Inhibitors of Amyloid β-Peptide Polymerization, Biochemistry, 1999, Vol.38, No.21, pages 6791 to 6800, particularly, abstract | 8 |
| Y | SOTO, C., β-amyloid disrupting drugs: Potential in the treatment of Alzheimer's disease., CNS Drugs, 1999, Vol.12, No.5, pages 347 to 356, particularly, abstract; Fig. 2 | 8 |
| Y | FINDEIS, Mark A. et al., Characterization of cholyl-leu-val-phephe-ala-OH as an inhibitor of amyloid beta-peptide polymerization, Amyloid, 11 December, 2001 (11.12.01), Vol.8, No.4, pages 231 to 241, particularly, abstract | 8 |
| Y | WO 98/08868 A1 (PRAESIS PHARMACEUTICALS INC.), 05 March, 1998 (05.03.98), Particularly, abstract; Claims<br>& EP 929574 A1 & US 6303567 B1<br>& US 5817626 A & US 5854215 A<br>& US 5985242 A & US 6277826 B1<br>& US 2002/103134 A1 & JP 2001-500852 A<br>& AU 9742387 A1 & AU 741199 B2 | 8 |
| Y | HOWLETT, David R. et al., Inhibition of fibril formation in β-amyloid peptide by a novel series of benzofurans, Biochemical Journal, 1999, Vol.340, No.1, pages 283 to 289, particularly, abstract; Fig. 2; table 1 | 8 |
| Y | HOWLETT, D.R. et al., Identification of a novel class of inhibitor of beta-amyloid peptide aggregation., British Journal of Pharmacology, 1998, Vol.123, No.PROC.SUPPL., p.25P. | 8 |

## INTERNATIONAL SEARCH REPORT

International application No.
PCT/JP02/13478

**Box I Observations where certain claims were found unsearchable (Continuation of item 2 of first sheet)**

This international search report has not been established in respect of certain claims under Article 17(2)(a) for the following reasons:

1. [X] Claims Nos.: 10, 11
   because they relate to subject matter not required to be searched by this Authority, namely:
   Claims 10, 11 pertain to methods for treatment of the human body by surgery or therapy and thus relates to a subject matter which this International Searching Authority is not required, under the provisions of Article 17(2)(a)(i) of the PCT and Rule 39.1(iv) of the Regulations under the PCT, to search.

2. [ ] Claims Nos.:
   because they relate to parts of the international application that do not comply with the prescribed requirements to such an extent that no meaningful international search can be carried out, specifically:

3. [ ] Claims Nos.:
   because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)